# EUROPEAN PATENT APPLICATION

(11) **EP 4 620 971 A1**
(43) Date of publication of application: **24.09.2025**
(21) Application number: 24305432.7
(22) Date of filing: 22.03.2024
(51) Int. Cl.: C07K 14/47, C07K 14/725, A61K 35/17

(54) **T-CELL RECEPTORS RECOGNIZING NEORIBOEPITOPES**

(71) Applicant: Ervimmune, 69008 Lyon (FR); Centre Léon Bérard, 69008 Lyon (FR); UNIVERSITE CLAUDE BERNARD - LYON 1, 69100 Villeurbanne (FR); Institut National de la Santé et de la Recherche Médicale, 75013 Paris (FR); Centre National de la Recherche Scientifique, 75016 Paris (FR)
(72) Inventor: DEPIL, Stéphane, 69008 LYON (FR); BAULU, Estelle, 69008 LYON (FR); CHUVIN, Nicolas, 69008 LYON (FR); GARDET, Célia, 69008 LYON (FR)
(74) Representative: Icosa

(57) **Abstract**

The present invention relates to an isolated T cell receptor (TCR) binding specifically to an epitope derived from non-canonical initiation and/or termination of translation of a gene of the *MYC* family, preferably *C-MYC.* The present invention also relates to a nucleic acid encoding said TCR, a vector comprising the nucleic acid, and a cell, preferably a T cell, comprising the same. Another aspect of the present invention is a cell, preferably a T cell, comprising said TCR for use as a medicament or for treating cancer.

## Description

### FIELD OF INVENTION

The present invention relates to an isolated T cell receptor (TCR) binding specifically to an epitope derived from non-canonical initiation and/or termination of translation of a gene of the *MYC* family, preferably *C-MYC.* The present invention also relates to a nucleic acid encoding said TCR, a vector comprising the nucleic acid, and a cell, preferably a T cell, comprising the same. Another aspect of the present invention is a cell, preferably a T cell, comprising said TCR for use as a medicament or for treating cancer.

### BACKGROUND OF INVENTION

T cell receptors (TCR) are receptors present on the surface of T cells, which recognize and bind with the complex formed by major histocompatibility complex (MHC) molecule and an epitope (MHC-epitope complex). There are two types of TCR: one type consisting of an alpha (α) chain and a beta (β) chain encoded by *TRA* and *TRB*, respectively (most frequently encountered type), and one type consisting of gamma (γ) and delta (δ) chains encoded by *TRG* and *TRD*, respectively (less frequently encountered type).

T cells comprising TCR (TCR-T cells) represent a promising therapeutic approach in cancer. Indeed, promising results have been obtained in clinical trials with TCR-T cells targeting different tumor antigen families, including tumor-associated antigens (*i*.*e*. antigens overexpressed in cancers but with a limited expression in normal tissues) and tumor-specific antigens (*i*.*e*. neoantigens exclusively expressed by tumor cells because they are linked to the tumorigenesis process). As an example, TCR-T cells targeting NY-ESO-1 showed promising results in clinical trials, especially in melanoma and synovial sarcoma, with an average response rate of 47% with 8 complete responses and 40 partial responses without major toxicities. As another example, clinical responses were reported in 17% of patients (2 of 12) without apparent toxicities with TCR-T cells targeting E6 viral antigen from human papilloma virus 16 (HPV16-E6).

In the present application, the Applicants have developed a new TCR targeting an epitope derived from non-canonical initiation and/or termination of translation of a gene of the *MYC* family, preferably *C-MYC.* In particular, the Applicants have found that the administration of T cells comprising said TCR was able to decrease the tumor volume in tumor animal models.

### SUMMARY

The present invention relates to an isolated T cell receptor (TCR) binding specifically to an epitope derived from non-canonical initiation and/or termination of translation of a gene of the *MYC* family, preferably *C-MYC.*

In one embodiment, the epitope as described hereinabove comprises or consists of a sequence with SEQ ID NO: 13.

In one embodiment, the TCR as described hereinabove comprises a α chain and a β chain, wherein the variable region of the α chain binds at least one of the following residues of SEQ ID NO: 13: Leu1, Leu2, Leu3, Glu4, and wherein the variable region of the β chain binds at least one of the following residues of SEQ ID NO: 13: Thr6, Ala7, Asn8.

In one embodiment, the TCR as described hereinabove comprises a α chain and a β chain,
- wherein the variable region of the α chain comprises at least one of the following CDRs:
   - CDR1 comprising or consisting of SEQ ID NO: 26,
   - CDR2 comprising or consisting of SEQ ID NO: 2, and/or
   - CDR3 comprising or consisting of SEQ ID NO: 27, or variants thereof having at least 90% of identity with SEQ ID NOs: 26, 2 and 27, and
- wherein the variable region of the β chain comprises at least one of the following CDRs:
   - CDR1 comprising or consisting of SEQ ID NO: 28,
   - CDR2 comprising or consisting of SEQ ID NO: 29, and/or
   - CDR3 comprising or consisting of SEQ ID NO: 30, or variants thereof having at least 90% of identity with SEQ ID NOs: 28-30.

In one embodiment, the TCR as described hereinabove comprises a α chain and a β chain,
- wherein the variable region of the α chain comprises the 3 following CDRs:
   - CDR1 comprising or consisting of SEQ ID NO: 26,
   - CDR2 comprising or consisting of SEQ ID NO: 2, and
   - CDR3 comprising or consisting of SEQ ID NO: 27, or variants thereof having at least 90% of identity with SEQ ID NOs: 26, 2 and 27, and
- wherein the variable region of the β chain comprises the 3 following CDRs:
   - CDR1 comprising or consisting of SEQ ID NO: 28,
   - CDR2 comprising or consisting of SEQ ID NO: 29, and
   - CDR3 comprising or consisting of SEQ ID NO: 30, or variants thereof having at least 90% of identity with SEQ ID NOs: 28-30.

In one embodiment, the TCR as described hereinabove comprises an α chain and a β chain,
- wherein the variable region of the α chain comprises the 3 following CDRs:
   - CDR1 consisting of SEQ ID NO: 1,
   - CDR2 consisting of SEQ ID NO: 2, and
   - CDR3 consisting of SEQ ID NO: 3, and
- wherein the variable region of the β chain comprises the 3 following CDRs:
   - CDR1 consisting of SEQ ID NO: 4,
   - CDR2 consisting of SEQ ID NO: 5, and
   - CDR3 consisting of SEQ ID NO: 6.

In one embodiment, the TCR as described hereinabove comprises a α chain and a β chain,
- wherein the variable region of the α chain comprises or consists of the sequence with SEQ ID NO: 14, or a variant thereof having at least 90% of identity with SEQ ID NO: 14, and
- wherein the variable region of the β chain comprises or consists of the sequence with SEQ ID NO: 15, or a variant thereof having at least 90% of identity with SEQ ID NO: 15.

In one embodiment, the TCR as described hereinabove comprises murine constant regions.

The present invention further relates to a nucleic acid encoding the TCR as described hereinabove.

In one embodiment, the nucleic acid as described hereinabove comprises:
- a sequence encoding the variable region of the α chain of the TCR with SEQ ID NO: 16, or a variant thereof having at least 70% of identity with SEQ ID NO: 16, and
- a sequence encoding the variable region of the β chain of the TCR with SEQ ID NO: 17, or a variant thereof having at least 70% of identity with SEQ ID NO: 17.

The present invention further relates to a vector comprising the nucleic acid as described hereinabove.

The present invention further relates to a cell comprising the TCR, the nucleic acid, or the vector as described herein, wherein preferably the cell is a T-cell, more preferably wherein the T cell is modified for silencing or genetically knocking out endogenous TCR chains.

The present invention further relates to the isolated TCR, the nucleic acid, the vector, or the cell as described hereinabove, for use as a medicament.

The present invention further relates to the isolated TCR, the nucleic acid, the vector, or the cell as described hereinabove, for use in treating a cancer in a subject in need thereof.

In one embodiment, the cancer is selected from the group comprising or consisting of breast cancer, including triple negative breast cancer, ovarian cancer, melanoma, sarcoma, teratocarcinoma, colon cancer, prostate cancer, bladder cancer, lung cancer, including non-small cell lung carcinoma and small cell lung carcinoma, head and neck cancer, colorectal cancer, glioblastoma, leukemias, lymphomas and other solid tumors and hematological malignancies.

### DEFINITIONS

In the present invention, the following terms have the following meanings:
**"Conservative amino acid modifications"** refers to modifications that do not significantly affect or alter the binding characteristics of a T cell receptor (TCR) containing the amino acid sequence. Such conservative modifications include amino acid substitutions, additions and deletions. Modifications can be introduced into TCRs by standard techniques known in the art, such as site-directed mutagenesis and PCR-mediated mutagenesis. Conservative amino acid substitutions are typically those in which an amino acid residue is replaced with an amino acid residue having a side chain with similar physicochemical properties. Where substitutions are made, preferred substitutions will be conservative modifications. Families of amino acid residues having similar side chains have been defined in the art. These families include amino acids with basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine, tryptophan), nonpolar side chains (*e.g.*, alanine, valine, leucine, isoleucine, proline, phenylalanine, methionine), β-branched side chains (*e.g.*, threonine, valine, isoleucine) and aromatic side chains (*e.g.*, tyrosine, phenylalanine, tryptophan, histidine).
**"Epitope"** refers to a specific arrangement of amino acids or sugar side chains located on a protein to which a TCR or a protein as described herein binds. Epitopes often consist of a chemically active surface grouping of molecules such as amino acids or sugar side chains, and have specific three-dimensional structural characteristics as well as specific charge characteristics. Epitopes can be linear (or sequential) or conformational, i.e., involving two or more sequences of amino acids in various regions that may not necessarily be contiguous.
**"Frameshift"** refers to a change in the open reading frame by one or more bases in either the 5' or 3' directions during translation.
**"Identity"** or **"identical",** when used herein in a relationship between the sequences of two or more amino acid sequences, or of two or more nucleic acid sequences, refers to the degree of sequence relatedness between amino acid sequences or nucleic acid sequences, as determined by the number of matches between strings of two or more amino acid residues or nucleic acid residues. "Identity" measures the percent of identical matches between the smaller of two or more sequences with gap alignments (if any) addressed by a particular mathematical model or computer program (i.e., "algorithms"). Identity of related amino acid sequences or nucleic acid sequences can be readily calculated by known methods. Such methods include, but are not limited to, those described in Lesk A. M. (1988). Computational molecular biology: Sources and methods for sequence analysis. New York, NY: Oxford University Press; Smith D. W. (1993). Biocomputing: Informatics and genome projects. San Diego, CA: Academic Press; Griffin A. M. & Griffin H. G. (1994). Computer analysis of sequence data, Part 1. Totowa, NJ: Humana Press; von Heijne G. (1987). Sequence analysis in molecular biology: treasure trove or trivial pursuit. San Diego, CA: Academic press; Gribskov M. R. & Devereux J. (1991). Sequence analysis primer. New York, NY: Stockton Press; Carillo et al., 1988. SIAM J Appl Math. 48(5): 1073-82. Preferred methods for determining identity are designed to give the largest match between the sequences tested. Methods of determining identity are described in publicly available computer programs. Preferred computer program methods for determining identity between two sequences include the GCG program package, including GAP (Genetics Computer Group, University of Wisconsin, Madison, WI; Devereux et al., 1984. Nucleic Acids Res. 12(1 Pt 1):387-95), BLASTP, BLASTN, and FASTA (Altschul et al., 1990. J Mol Biol. 215(3):403-10). The BLASTX program is publicly available from the National Center for Biotechnology Information (NCBI) and other sources (BLAST Manual, Altschul et al. NCB/NLM/NIH Bethesda, Md. 20894). The well-known Smith Waterman algorithm may also be used to determine identity.
**"Mammal"** refers to any mammal, including humans, non-human primates, domestic and farm animals, and zoo, sports, or pet animals, such as dogs, cats, cattle, horses, sheep, pigs, goats, rabbits, monkeys, etc. Preferably, the mammal is human.
**"Monocistronic"** means that a single nucleic acid is expressed in a single vector.
**"Open reading frame"** or **"ORF"** refers to a sequence of nucleotide triplets that code for amino acids located between an initiation codon and a stop codon in the same reading frame.
**"Pharmaceutically acceptable excipient"** includes any and all solvents, dispersion media, coatings, antibacterial and antifungal agents, isotonic and absorption delaying agents and the like. Said excipient does not produce an adverse, allergic or other untoward reaction when administered to an animal, preferably a mammal, more preferably a human. For human administration, preparations should meet sterility, pyrogenicity, and general safety and purity standards as required by regulatory offices, such as, for example, FDA Office or EMA.
**"Polycistronic"** means that at least two or more nucleic acids are expressed in a single vector.
**"Readthrough"** refers to a process wherein, in translation, a stop codon is interpreted as a sense codon.
**"Subject"** refers to a mammal, preferably a human. A subject may be a "*patient*", *i.e.*, a warm-blooded animal, more preferably a human, who/which is awaiting the receipt of, or is receiving medical care or was/is/will be the object of a medical procedure, or is monitored for the development of a disease. The term "mammal" refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.
**"Treatment"** refers to a therapeutic (or curative) treatment, to a prophylactic (or preventive) treatment, or to both a therapeutic (or curative) treatment and a prophylactic (or preventive) treatment, wherein the object is to prevent, reduce, slow down (lessen), or cure one or more of the symptom(s) or manifestation(s) of the cancer as described herein.
**"Therapeutically effective amount"** refers to the level or amount of a TCR, a protein, a nucleic acid, a vector, a cell, or a population of cells as described herein that is aimed at, without causing significant negative or adverse side effects to the target, (1) delaying or preventing the onset of a disease, disorder, or condition; (2) slowing down or stopping the progression, aggravation, or deterioration of one or more symptoms of the disease, disorder, or condition; (3) bringing about ameliorations of the symptoms of the disease, disorder, or condition; (4) reducing the severity or incidence of the disease, disorder, or condition; or (5) curing the disease, disorder, or condition. A therapeutically effective amount may be administered prior to the onset of the disease, disorder, or condition, for a prophylactic or preventive action. Alternatively or additionally, the therapeutically effective amount may be administered after initiation of the disease, disorder, or condition, for a therapeutic action.
**"Vector"** refers to the vehicle by which a DNA or RNA sequence (*e.g.* a foreign gene) can be introduced into a host cell, so as to transform a host and promote expression (*e*.*g*. transcription and translation) of the introduced sequence.

### DETAILED DESCRIPTION

This invention relates to a T cell receptor (TCR) binding specifically to an epitope derived from non-canonical initiation and/or termination of translation of a gene of the *MYC* family, preferably *C-MYC.*

Myc family refers to a family of oncogenes coding for proteins, and in particular, the transcription factors c-myc, 1-myc and n-myc, which are encoded by *C-MYC* gene, *MYCL* gene and *MYCN* gene in humans, respectively. *C-MYC* refers to the proto-oncogene coding for the transcription factor c-myc which is involved in nucleic acid metabolism and in mediating the cellular response to growth factors. Truncation of the first exon of the gene, which appears to regulate c-myc expression, is crucial for tumorigenicity. The human gene coding for c-myc is located at 8q24 on the long arm of chromosome 8.

As used herein, "binding specifically" means that the TCR is specific for an epitope derived from non-canonical initiation and/or termination of translation of a gene of the *MYC* family, preferably *C-MFC* and exhibits low or no binding with other epitopes.

Examples of methods for assessing the binding specificity of TCRs are described in the Example part. For example, binding specificity may be assessed by multiparametric flow cytometry, or functional analysis (*e*.*g*. measure of 4-1BB expressing cells, measure of expression of IFNγ, TNF, IL-2 and Granzyme B).

The epitope is derived from non-canonical initiation and/or termination of translation of a gene of the *MFC* family, preferably *C-MYC.*

As used herein, "non-canonical initiation and/or termination of translation of a gene" refers to non-conventional mechanisms of translation initiation and/or termination, which may be induced under stress conditions such as, for example, hypoxia, apoptosis, starvation, and viral infection. The conventional mechanism for translation initiation involves recruitment of the 40S ribosome to the cap structure at the 5' end of the mRNA, followed by linear scanning of the 5'-UTR until the initiation codon is reached. The non-canonical mechanisms of initiation and/or termination of translation of a gene includes, without limitation, the following events: frameshifts, readthroughs, translation of regions on 5'UTR and/or 3'UTR that are normally non translated (e.g. initiation of translation upstream of the initiation codon, termination of translation downstream of the stop codon, and/or initiation of translation occurring downstream of the stop codon of the coding sequence), and IRES-dependent initiation of translation.

As used herein, "Internal ribosome entry site" or "IRES" are sequences that can recruit ribosomes and allow translation.

Examples of mechanisms of non-canonical initiation and/or termination of translation of a given gene include, without limitation, frameshifts of the open reading frame, stop codon readthroughs, translation of 5'UTR and/or 3'UTR, or IRES-dependent initiation of translation.

By "translation of 5'UTR and/or 3'UTR", it is meant that the 5'UTR and/or the 3'UTR of a given mRNA that are normally non-translated in a canonical translation, are translated. Translation of 5'UTR means that the initiation of translation starts upstream of the initiation codon. Translation of 3'UTR means that the termination of translation stops downstream of the stop codon and/or that initiation of translation occurs downstream of the stop codon of the coding sequence. The translation of the 5'UTR and/or 3'UTR may be a complete or a partial translation of the 5'UTR and/or 3'UTR.

The epitope derived from non-canonical initiation and/or termination of translation of a gene of the *MYC* family, preferably *C-MFC* as described herein may comprise or consist of the sequence with SEQ ID NO: 13 or a variant thereof having a sequence that shares at least 70%, 75%, 80%, 85%, 90%, 91%, 92%, 93%, 94%, 95%, 96%, 97%, 98%, 99% or more of identity with SEQ ID NO: 13.

An example of an epitope consisting of the sequence with SEQ ID NO: 13 is PR3 .

As used herein, "PR3" refers to an epitope derived from non-canonical initiation and/or termination of translation of *C-MYC*, consisting of the sequence SEQ ID NO: 13.

The TCR as described herein may bind a complex comprising an epitope as described herein and a major histocompatibility complex (MHC) molecule.

The MHC gene family is divided into three subgroups: MHC class I, MHC class II, and MHC class III. Thus, the complex as described herein may comprise an epitope as described herein and a MHC class I molecule, a MHC class II molecule, or a MHC class III molecule, preferably a MHC class I molecule.

In humans, the MHC class I comprises HLA-A, HLA-B, and HLA-C molecules. Thus, the complex as described herein may comprise an epitope as described herein and a HLA-A molecule, including HLA-A2 molecule, a HLA-B molecule or a HLA-C molecule, preferably a HLA-A molecule, more preferably a HLA-A2 molecule.

The TCR as described herein may be isolated. As used herein, the term "isolated TCR" means that the TCR is substantially separated from other proteins as well as cell membrane or other components of a cell. The TCR may be obtained by removal from its natural environment or chemical synthesis.

The TCR as described herein may be with a similar structure as a TCR occurring in natural environment. The TCR as described herein may be with a structure not occurring in natural environment.

The TCR as described herein may be functional, *i.e.* able to active T cell function when binding a complex comprising an epitope as described herein and a major histocompatibility complex (MHC) molecule.

Means for assessing the functionality of TCRs and of cells expressing the TCRs at the cell surface are presented in the Example part, and include, without limitation, assessing expression of activation markers (*e*.*g*. 4-1BB or CD69), assessing secretion of cytokines (*e*.*g*. IFNγ, TNF, IL-2, Granzyme B), and assessing cell cytolysis.

The TCR as described herein may comprise two chains selected among α chains, β chains, γ chains, δ chains and combinations thereof.

The TCR as described herein may comprise or consist of a combination of a α chain and a β chain, or a combination of a γ chain and a δ chain, preferably a combination of a α chain and a β chain.

Each chain of the α and β chains is composed of two extracellular regions: one variable region, which contains 3 complementarity-determining regions (CDRs) and enables the binding with the MHC-epitope complex, and one constant region.

The TCR as described herein may comprise human variable regions. In particular, the TCR as described herein may comprise a human α chain variable region and/or a human β chain variable region.

As used herein, the term "human variable region" means that the variable region is derived from the α chain and/or the β chain of a TCR derived from a human, *i.e.* a TCR that was expressed in a human T cell.

The variable region of the α chain of the TCR as described herein may bind at least one of the following residues of SEQ ID NO: 13: Leu1, Leu2, Leu3, Glu4. The variable region of the α chain of the TCR as described herein may bind the residues Leu1, Leu2, Leu3, and Glu4 of SEQ ID NO: 13.

The variable region of the β chain of the TCR as described herein may bind at least one of the following residues of SEQ ID NO: 13: Thr6, Ala7, Asn8. The variable region of the β chain of the TCR as described herein may bind the residues Thr6, Ala7, and Asn8 of SEQ ID NO: 13.

The TCR as described herein may comprise a α chain and a β chain, wherein the variable region of the α chain binds at least one of the following residues of SEQ ID NO: 13: Leu1, Leu2, Leu3, Glu4, and wherein the variable region of the β chain binds at least one of the following residues of SEQ ID NO: 13: Thr6, Ala7, Asn8.

The TCR as described herein may comprise a α chain and a β chain, wherein the variable region of the α chain binds the residues Leu1, Leu2, Leu3 and Glu4 of SEQ ID NO: 13, and wherein the variable region of the β chain binds the residues Thr6, Ala7, and Asn 8 of SEQ ID NO: 13.

The CDRs of the TCR as described herein may be determined by methods known to the skilled artisan in the art.

The CDRs of the TCR as described herein may be determined by IgBLAST.

The variable region of the α chain of the TCR as described herein may comprise at least one the following CDRs (*e*.*g*., 1, 2 or 3):
- CDR1 comprising or consisting of SEQ ID NO: 26,
- CDR2 comprising or consisting of SEQ ID NO: 2, and/or
- CDR3 comprising or consisting of SEQ ID NO: 27.

The variable region of the α chain of the TCR as described herein may comprise the 3 following CDRs:
- CDR1 comprising or consisting of SEQ ID NO: 26,
- CDR2 comprising or consisting of SEQ ID NO: 2, and
- CDR3 comprising or consisting of SEQ ID NO: 27.

The variable region of the β chain of the TCR as described herein may comprise at least one the following CDRs (*e*.*g*., 1, 2 or 3):
- CDR1 comprising or consisting of SEQ ID NO: 28,
- CDR2 comprising or consisting of SEQ ID NO: 29, and/or
- CDR3 comprising or consisting of SEQ ID NO: 30.

The variable region of the β chain of the TCR as described herein may comprise the 3 following CDRs:
- CDR1 comprising or consisting of SEQ ID NO: 28,
- CDR2 comprising or consisting of SEQ ID NO: 29, and
- CDR3 comprising or consisting of SEQ ID NO: 30.

The TCR as described herein may comprise a α chain and a β chain,
- wherein the variable region of the α chain comprises at least one of the following CDRs (*e.g.*, 1, 2 or 3):
   - CDR1 comprising or consisting of SEQ ID NO: 26,
   - CDR2 comprising or consisting of SEQ ID NO: 2, and/or
   - CDR3 comprising or consisting of SEQ ID NO: 27, and
- wherein the variable region of the β chain comprises at least one of the following CDRs (*e*.*g*., 1, 2 or 3):
   - CDR1 comprising or consisting of SEQ ID NO: 28,
   - CDR2 comprising or consisting of SEQ ID NO: 29, and/or
   - CDR3 comprising or consisting of SEQ ID NO: 30.

The TCR as described herein may comprise a α chain and a β chain,
- wherein the variable region of the α chain comprises the 3 following CDRs (*e.g.*, 1, 2 or 3):
   - CDR1 comprising or consisting of SEQ ID NO: 26,
   - CDR2 comprising or consisting of SEQ ID NO: 2, and
   - CDR3 comprising or consisting of SEQ ID NO: 27, and
- wherein the variable region of the β chain comprises the 3 following CDRs (*e*.*g*., 1, 2 or 3):
   - CDR1 comprising or consisting of SEQ ID NO: 28,
   - CDR2 comprising or consisting of SEQ ID NO: 29, and
   - CDR3 comprising or consisting of SEQ ID NO: 30.

The CDRs of the TCR as described herein may be determined by the online TCRpMHCmodels - 1.0 tool.

The variable region of the α chain of the TCR as described herein may comprise at least one the following CDRs (*e*.*g*., 1, 2 or 3):
- CDR1 consisting of SEQ ID NO: 1,
- CDR2 consisting of SEQ ID NO: 2, and/or
- CDR3 consisting of SEQ ID NO: 3.

The variable region of the α chain of the TCR as described herein may comprise the 3 following CDRs:
- CDR1 consisting of SEQ ID NO: 1,
- CDR2 consisting of SEQ ID NO: 2, and
- CDR3 consisting of SEQ ID NO: 3.

The variable region of the β chain of the TCR as described herein may comprise at least one the following CDRs (*e*.*g*., 1, 2 or 3):
- CDR1 consisting of SEQ ID NO: 4,
- CDR2 consisting of SEQ ID NO: 5, and/or
- CDR3 consisting of SEQ ID NO: 6.

The variable region of the β chain of the TCR as described herein may comprise the 3 following CDRs:
- CDR1 consisting of SEQ ID NO: 4,
- CDR2 consisting of SEQ ID NO: 5, and
- CDR3 consisting of SEQ ID NO: 6.

The TCR as described herein may comprise a α chain and a β chain,
- wherein the variable region of the α chain comprises at least one of the following CDRs (*e.g.*, 1, 2 or 3):
   - CDR1 consisting of SEQ ID NO: 1,
   - CDR2 consisting of SEQ ID NO: 2, and/or
   - CDR3 consisting of SEQ ID NO: 3, and
- wherein the variable region of the β chain comprises at least one of the following CDRs (*e.g.*, 1, 2 or 3):
   - CDR1 consisting of SEQ ID NO: 4,
   - CDR2 consisting of SEQ ID NO: 5, and/or
   - CDR3 consisting of SEQ ID NO: 6.

The TCR as described herein may comprise an α chain and a β chain,
- wherein the variable region of the α chain comprises the 3 following CDRs:
   - CDR1 consisting of SEQ ID NO: 1,
   - CDR2 consisting of SEQ ID NO: 2, and
   - CDR3 consisting of SEQ ID NO: 3, and
- wherein the variable region of the β chain comprises the 3 following CDRs:
   - CDR1 consisting of SEQ ID NO: 4,
   - CDR2 consisting of SEQ ID NO: 5, and
   - CDR3 consisting of SEQ ID NO: 6.

The TCR as described herein may comprise variants of any of CDR1, CDR2 and/or CDR3 of the α chain with SEQ ID NOs: 1-3 or SEQ ID NOs: 26, 2 and 27. The TCR as described herein may comprise variants of any of CDR1, CDR2 and/or CDR3 of the β chain with SEQ ID NOs: 4-6 or SEQ ID NOs: 28-30.

Variants of CDR1, CDR2 and/or CDR3 of the α chain and/or of the β chain as described herein may be functional variants, *i.e.* variants that retain the ability to bind an epitope as described herein.

Variants of CDR1, CDR2 and/or CDR3 of the α chain and/or of the β chain as described herein may have 1, 2, 3 or more conservative amino acid modifications, such as insertions, deletions and/or substitutions.

In particular, one or more amino acid residues within the CDRs of the TCR as described herein may be replaced with other amino acid residues from the same side chain family and the altered TCR can be tested for retained function (i.e., the properties set forth herein, such as, e.g., the binding to an epitope derived from non-canonical initiation and/or termination of translation of a gene of the *MYC* family, preferably *C-MYC*) using the methods as exemplified herein. A string of amino acids within the CDRs of the TCR can be replaced with a structurally similar string that differs in order and/or composition of side chain family members.

Variants of CDR1, CDR2 and/or CDR3 of the α chain and/or of the β chain as described herein may have an amino acid sequence that shares at least 70%, 75%, 80%, 85%, 90%, 91%, 92%, 93%, 94%, 95%, 96%, 97%, 98%, 99% or more of identity with the particular CDR or sets of CDRs listed in the corresponding SEQ ID NOs.

Variants of CDR1, CDR2 and/or CDR3 of the α chain and/or of the β chain as described herein may have an amino acid sequence that shares at least 90% or more of identity with the particular CDR or sets of CDRs listed in the corresponding SEQ ID NOs.

The variable region of the α chain of the TCR as described herein may comprise or consist of the sequence with SEQ ID NO: 14.

The variable region of the β chain of the TCR as described herein may comprise or consist of the sequence with SEQ ID NO: 15.

The TCR as described herein may comprise a α chain and a β chain,
- wherein the variable region of the α chain comprises or consists of the sequence with SEQ ID NO: 14, and
- wherein the variable region of the β chain comprises or consists of the sequence with SEQ ID NO: 15.

The TCR as described herein may comprise variants of the variable region of the α chain with SEQ ID NO: 14. The TCR as described herein may comprise variants of the variable region of the β chain with SEQ ID NO: 15.

Variants of the variable regions of the α chain and/or of the β chain as described herein may be functional variants, *i.e.* variants that retain the ability to bind an epitope as described herein.

Variants of the variable regions of the α chain and/or of the β chain as described herein may have 1, 2, 3 or more conservative amino acid modifications, such as insertions, deletions and/or substitutions.

In particular, one or more amino acid residues within the variable regions of the TCR as described herein may be replaced with other amino acid residues from the same side chain family and the altered TCR can be tested for retained function (i.e., the properties set forth herein, such as, *e*.*g*., the binding to an epitope derived from non-canonical initiation and/or termination of translation of a gene of the *MYC* family, preferably *C-MYC*) using the methods as exemplified herein. A string of amino acids within the variable regions of the TCR can be replaced with a structurally similar string that differs in order and/or composition of side chain family members.

Variants of the variable regions of the α chain and/or of the β chain as described herein may have an amino acid sequence that shares at least 70%, 75%, 80%, 85%, 90%, 91%, 92%, 93%, 94%, 95%, 96%, 97%, 98%, 99% or more of identity with SEQ ID NOs: 14 and 15, respectively.

Variants of the variable regions of the α chain and/or of the β chain as described herein may have an amino acid sequence that shares at least 90% or more of identity with SEQ ID NOs: 14 and 15, respectively.

Variants of the variable regions as described herein may comprise amino acid modifications, preferably conservative amino acid modifications, outside the CDRs (*e.g.* in the framework regions).

The TCR as described herein may comprise murine constant regions. In particular, the TCR as described herein may comprise a murine α chain constant region and/or a murine β chain constant region.

As used herein, the term "murine constant region" means that the constant region is derived from the α chain and/or the β chain of a TCR derived from a mouse, *i.e.* a TCR that was expressed in a mouse T cell.

The TCR as described herein may comprise a α chain and/or a β chain with human variable regions as described herein and murine constant regions. In particular, the TCR as described herein may comprise a α chain and a β chain with human variable regions as described herein and murine constant regions. Thus, the TCR as described herein may be a chimeric mouse-human TCR.

An example of a murine constant region of α chain is SEQ ID NO: 18. An example of a murine constant region of β chain is SEQ ID NO: 19.

Thus, the TCR as described herein may comprise a α chain comprising or consisting of the sequence with SEQ ID NO: 18 and/or a β chain comprising or consisting of the sequence with SEQ ID NO: 19.

The TCR as described herein may comprise human constant regions. In particular, the TCR as described herein may comprise a human α chain constant region and/or a human β chain constant region.

As used herein, the term "human constant region" means that the constant region is derived from the α chain and/or the β chain of a TCR derived from a human, *i.e.* a TCR that was expressed in a human T cell.

The TCR as described herein may comprise a α chain and/or a β chain with human variable regions as described herein and human constant regions. In particular, the TCR as described herein may comprise a α chain and a β chain with human variable regions as described herein and human constant regions. Thus, the TCR as described herein may be a fully human TCR.

An example of a human constant region of α chain is SEQ ID NO: 22. An example of a human constant region of β chain is SEQ ID NO: 23.

Thus, the TCR as described herein may comprise a α chain comprising or consisting of the sequence with SEQ ID NO: 22 and/or a β chain comprising or consisting of the sequence with SEQ ID NO: 23.

The TCR as described herein may comprise variants of the constant region of the α chain with SEQ ID NO: 18 or 22. The TCR as described herein may comprise variants of the variable region of the β chain with SEQ ID NO: 19 or 23.

Variants of the constant regions of the α chain and/or of the β chain as described herein may have an amino acid sequence that shares at least 70%, 75%, 80%, 85%, 90%, 91%, 92%, 93%, 94%, 95%, 96%, 97%, 98%, 99% or more of identity with SEQ ID NO: 18 or 22 and SEQ ID NO: 19 or 23, respectively.

Variants of the constant regions of the α chain and/or of the β chain as described herein may have 1, 2, 3 or more amino acid modifications, such as insertions, deletions and/or substitutions with respect to SEQ ID NO: 18 or 22 and SEQ ID NO: 19 or 23, respectively.

Variants of the constant regions of the α chain and/or of the β chain as described herein may have one or the two following modifications:
- the substitution of the threonine residue in the motif DKTVL (SEQ ID NO: 31) with a cysteine residue in the constant region of the α chain, and/or
- the substitution of the serine residue in the motif GVSTD (SEQ ID NO: 32) with a cysteine residue in the constant region of the β chain.

Variants of the constant regions of the α chain and/or of the β chain as described herein may have one or the two following modifications:
- the substitution T47C (*i.e.* the substitution of the threonine at position 47 with a cysteine residue) with respect to SEQ ID NO: 18 or SEQ ID NO: 22, and/or
- the substitution S57C (*i.e.* the substitution of the serine at position 57 with a cysteine residue) with respect to SEQ ID NO: 19 or SEQ ID NO: 23.

An example of a variant of a murine constant region of α chain is SEQ ID NO: 20. An example of a variant of a murine constant region of β chain is SEQ ID NO: 21.

Thus, the TCR as described herein may comprise a α chain comprising or consisting of the sequence with SEQ ID NO: 20 and/or a β chain comprising or consisting of the sequence with SEQ ID NO: 21.

An example of a variant of a human constant region of α chain is SEQ ID NO: 24. An example of a variant of a human constant region of β chain is SEQ ID NO: 25.

Thus, the TCR as described herein may comprise a α chain comprising or consisting of the sequence with SEQ ID NO: 24 and/or a β chain comprising or consisting of the sequence with SEQ ID NO: 25.

The present invention also relates to a protein comprising a functional portion of the TCR as described herein.

As used herein, "functional portion" refers to a part of the TCR that retains the biological activity of the TCR, in particular, its ability to bind specifically the epitope as described herein. Thus, the "functional portion of a TCR" comprises the CDRs of the TCR as described herein.

The protein as described herein may be isolated. As used herein, the term "isolated protein" means that the protein is substantially separated from other proteins as well as cell membrane or other components of a cell. The protein may be obtained by removal from its natural environment or chemical synthesis.

The protein as described herein may comprise a functional portion of the α chain, a functional portion of the β chain or a functional portion of both α and β chains of the TCR as described herein.

The protein as described herein may comprise all or part of the variable region of the α chain, of the β chain or of both α and β chains of the TCR as described herein. In particular, the protein as described herein may comprise all the variable regions of both α and β chains of the TCR as described herein.

The protein as described herein may be a soluble fragment of the TCR as described herein which contains all or part of the variable regions of the α and β chains of the TCR as described herein. Said fragment of TCR is not anchored at the cell surface and retains its ability to bind specifically the epitope as described herein.

The TCR or the protein as described herein may be modified by engineering techniques.

The TCR or the protein as described herein may be modified to improve its expression, its avidity and/or its affinity to the epitope. Means for improving expression and/or avidity and/or its affinity include, for example, the substitution of human constant regions with the corresponding murine constant regions, the addition of disulfide bonds, the addition of hydrophobic mutations, or the substitution of one or more amino acid(s) in the variable region allowing to conserve or improve the specificity for the epitope (see, for example, Rath and Arber, Cells. 2020 Jun; 9(6): 1485, and Baulu et al. Sci Adv. 2023 Feb 15;9(7):eadf3700, which are herein incorporated by reference).

The TCR has described herein may be modified to improve T cell function. Means for improving T cell function include, without limitation the substitution, addition and/or deletion of one or more amino acid(s) in the constant region allowing to improve downstream signaling, T cell assembling and/or T cell clustering.

In particular, the TCR or the protein as described herein may be modified to substitute the human constant regions of both α and β chains of the TCR with the corresponding murine constant regions. Such substitution may enable to avoid mispairing with endogenous TCRs.

The present invention also relates to a nucleic acid encoding the TCR or the protein as described herein.

The nucleic acid as described herein may be isolated. As used herein, the term "isolated nucleic acid" refers to a nucleic acid sequence that is substantially separated from other genome DNA sequences as well as proteins or complexes such as ribosomes and polymerases. The nucleic acid may be obtained by removal from its natural environment or chemical synthesis.

The nucleic acid as described herein may comprise a sequence encoding the variable region of the α chain of the TCR as described herein. An example of a sequence encoding the variable region of the α chain of the TCR as described herein is SEQ ID NO: 16.

Thus, the nucleic acid as described herein may comprise a nucleic acid sequence with SEQ ID NO: 16.

The nucleic acid as described herein may comprise a sequence encoding the variable region of the β chain of the TCR as described herein. An example of a sequence encoding the variable region of the β chain of the TCR as described herein is SEQ ID NO: 17.

Thus, the nucleic acid as described herein may comprise a nucleic acid sequence with SEQ ID NO: 17.

The nucleic acid as described herein may comprise a sequence encoding the variable region of the α chain of the TCR as described herein, and a sequence encoding the variable region of the β chain of the TCR as described herein.

The nucleic acid as described herein may comprise:
- a sequence encoding the variable region of the α chain of the TCR as described herein with SEQ ID NO: 16, and
- a sequence encoding the variable region of the β chain of the TCR as described herein with SEQ ID NO: 17.

The nucleic acid as described herein may comprise variants of the nucleic acid sequence with SEQ ID NO: 16. The nucleic acid as described herein may comprise variants of the nucleic acid sequence with SEQ ID NO: 17.

Variants of the nucleic acid sequences encoding for the variable regions of the α chain and/or of the β chain as described herein may be functional variants, *i.e.* variants encoding for variable regions retaining their ability to bind the epitope as described herein.

Variants of the nucleic acid sequences encoding for the variable regions of the α chain and/or of the β chain as described herein may have an amino acid sequence that shares at least 70%, 75%, 80%, 85%, 90%, 91%, 92%, 93%, 94%, 95%, 96%, 97%, 98%, 99% or more of identity with SEQ ID NOs: 16 and 17, respectively.

Variants of the nucleic acid sequences encoding for the variable regions of the α chain and/or of the β chain as described herein may have an amino acid sequence that shares at least 70% or more of identity with SEQ ID NOs: 16 and 17, respectively.

Variants of the nucleic acid sequences encoding for the variable regions as described herein may comprise nucleotide modifications outside the nucleic acid sequences coding for the CDRs.

The nucleic acid as described herein may comprise a sequence encoding the constant region of the α chain of the TCR as described herein, and a sequence encoding the constant region of the β chain of the TCR as described herein.

The nucleic acid as described herein may comprise sequences encoding human constant regions. The nucleic acid as described herein may comprise sequences encoding murine constant regions.

The present invention further relates to a vector comprising a nucleic acid as described herein.

A vector as described herein may comprise regulatory elements, such as a promoter, enhancer, terminator and the like, to cause or direct expression of said TCR or protein upon administration to a host. Examples of promoters and enhancers used in the expression vector for animal cell include early promoter and enhancer of SV40, LTR promoter and enhancer of Moloney mouse leukemia virus, promoter and enhancer of immunoglobulin H chain and the like. Any expression vector for animal cell can be used, so long as a gene encoding the TCR or the protein can be inserted and expressed.

Examples of suitable vectors include pAGE107, pAGE103, pHSG274, pKCR, pSG1 beta d2-4 and the like. Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found in the art.

The vector as described herein may be monocistronic. The vector as described herein may be polycistronic.

The present invention further relates to a cell comprising a TCR, a protein, a nucleic acid or a vector as described herein. The present invention further relates to a population of cells as described herein.

The cells as described herein may express the TCR or the protein at the cell surface. Thus, the present invention further relates to a cell expressing, preferably expressing at the cell surface, a TCR or a protein as described herein.

Means for assessing the expression of the TCRs at the cell surface are presented in the Example part, and include, without limitation, flow cytometry with dextramers.

The cell as described herein may be prokaryote, yeast, or eukaryote cells, preferably mammalian cells, such as, for example: monkey kidney CV1 line transformed by SV40 (COS-7, ATCC CRL 1651); human embryonic kidney line (293 or 293 cells subcloned for growth in suspension culture); baby hamster kidney cells (BHK, ATCC CCL 10); Chinese hamster ovary cells/-DHFR (CHO); mouse Sertoli cells (TM4); mouse myeloma cells SP2/0-AG14 (ATCC CRL 1581 ; ATCC CRL 8287) orNSO (HPA culture collections no. 85110503); monkey kidney cells (CVl ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells; MRC 5 cells; FS4 cells; and a human hepatoma line (Hep G2), as well as DSM's PERC-6 cell line. Expression vectors suitable for use in each of these cells are also generally known in the art.

The cell as described herein may be a T cell, preferably a human T-cell.

The T cell as described herein may be a cultured T cell (*e.g.* a primary T cell), a T cell from a cultured T cell line (*e.g.* Jurkat, SupT1) or a T cell obtained from a mammal, preferably a human.

The T cell as described herein may be a CD4+ T cell (helper T cell), a CD8+ T cell (cytotoxic T cell) or a regulatory T cell.

The cells as described herein may be modified to improve expression and/or avidity of the TCR as described herein. Means for improving expression and/or avidity of the TCR as described herein include, for example, silencing or genetically knocking out endogenous TCR chains (see Rath and Arber, and Baulu et al. supra).

In particular, the T cells as described herein may be modified for silencing or genetically knocking out endogenous TCR chains, such as, for example, using CRISPR/CAS9 technology or derivatives thereof.

As used herein, "CRISPR/CAS9 technology or derivatives thereof' includes any technology derived from CRISPR/CAS9, and in particular, technologies using other Cas proteins, such as, for example, Cas9 orthologs, Cas12, Cas13 and Cas14.

The present invention also relates to a composition comprising, consisting or consisting essentially of a TCR, a protein, a nucleic acid, a vector, a cell or a population of cells as described herein.

As used herein, "consisting essentially of', with reference to a composition, means that the TCR, the protein, the nucleic acid, the vector, the cell or the population of cells is the only one therapeutic agent or agent with a biologic activity within said composition.

The present invention also relates to a pharmaceutical composition comprising, consisting or consisting essentially of a TCR, a protein, a nucleic acid, a vector, a cell or a population of cells as described herein, and a pharmaceutically acceptable excipient.

The present invention also relates to a medicament comprising, consisting or consisting essentially of a TCR, a protein, a nucleic acid, a vector, a cell or a population of cells as described herein.

Examples of pharmaceutically acceptable excipients that may be used in the compositions of the present invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

The pharmaceutical compositions as described herein may comprise vehicles which are pharmaceutically acceptable for a formulation capable of being injected to a subject. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

For use in administration to a subject, the TCR, the protein, the nucleic acid, the vector, the cell, the population of cells, the composition, the pharmaceutical composition, or the medicament as described herein is to be formulated for administration to the subj ect.

The TCR, the protein, the nucleic acid, the vector, the cell, the population of cells, the composition, the pharmaceutical composition, or the medicament as described herein may be administered parenterally, by inhalation spray, rectally, nasally, or via an implanted reservoir. The term administration used herein includes subcutaneous, intravenous, intramuscular, intra-articular, intra-synovial, intrasternal, intrathecal, intrahepatic, intralesional and intracranial injection or infusion techniques.

The present invention also relates to a TCR, a protein, a nucleic acid, a vector, a cell or a population of cells as described herein for use as a medicament.

The present invention also relates to a TCR, a protein, a nucleic acid, a vector, a cell, a population of cells, a composition, a pharmaceutical composition, or a medicament as described herein for use in treating a cancer in a subject in need thereof.

The present invention also relates to a TCR, a protein, a nucleic acid, a vector, a cell, or a population of cells as described herein for use in the manufacture of a medicament for treating a cancer in a subject in need thereof.

The present invention also relates to a method for treating a cancer in a subject in need thereof, comprising administering to said subject a TCR, a protein, a nucleic acid, a vector, a cell, a population of cells, a composition, a pharmaceutical composition, or a medicament as described herein.

The TCR, the protein, the nucleic acid, the vector, the cell, or the population of cells as described herein may be administered at a therapeutically effective amount.

It will be however understood that the total daily usage of the TCR, the protein, the nucleic acid, the vector, the cell, the population of cells, the composition, the pharmaceutical composition, or the medicament as described herein will be decided by the attending physician within the scope of sound medical judgment.

The specific dose for any particular subject will depend upon a variety of factors including the symptom being treated and the severity of the symptom; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific compounds employed; and like factors well known in the medical arts.

The cancer as described herein may be selected from the group comprising or consisting of breast cancer, including triple negative breast cancer, ovarian cancer, melanoma, sarcoma, teratocarcinoma, colon cancer, prostate cancer, bladder cancer, lung cancer, including non-small cell lung carcinoma and small cell lung carcinoma, head and neck cancer, colorectal cancer, glioblastoma, leukemias, lymphomas and other solid tumors and hematological malignancies.

In particular, the cancer may be selected from the group comprising or consisting of breast cancer, ovarian cancer and colon cancer. The cancer may be a breast cancer, in particular a triple negative breast cancer. The cancer may be an ovarian cancer. The cancer may be a colon cancer.

Without being bound by a particular theory or mechanism, it is believed that since an epitope derived from non-canonical initiation and/or termination of translation of a gene of the *MYC* family as described herein, such as PR3, is specifically expressed in cancer cells, a TCR as described herein is able to target cancer cells expressing said epitope, and a T cell comprising said TCR is able to kill cancer cells expressing said epitope.

In particular, the TCR or the cell, such as the T cell, as described herein may display one or several of the following characteristics.

The TCR as described herein may be able to bind the complex formed by PR3 and a MHC molecule.

The T cell comprising a TCR as described herein may be able to be activated upon the presence of cells expressing the epitope recognized by the TCR (*e.g.* PR3).

The T cell comprising a TCR as described herein may be able to induce secretion of IFNγ, TNF, IL-2 and/or Granzyme B upon the presence of cells expressing the epitope recognized by the TCR (*e.g.* PR3).

The T cell comprising a TCR as described herein may be able to kill cancer cells expressing the epitope recognized by the TCR (*e.g.* PR3).

The T cell comprising a TCR as described herein may be able to spare normal cells (*i.e.* cells that do not express the epitope recognized by the TCR (*e.g.* PR3)).

The T cell comprising a TCR as described herein may be able to decrease tumor growth. The T cell comprising a TCR as described herein may be able to decrease tumor volume.

### SEQUENCE LISTING

| **SEQ ID NO:** | **Sequence** |
|---|---|
| 1 | YSDRGSQSF |
| 2 | IYSNGD |
| 3 | CAVTNAGKSTF |
| 4 | SPRSGDLSV |
| 5 | QYYNGEERAKGNILERFS |
| 6 | CASSFPLGGGRVDTQYF |
| 7 | TACAGTGACCGAGGTTCCCAGTCCTTC |
| 8 | ATATACTCCAATGGTGAC |
| 9 | TGTGCCGTTACGAATGCAGGCAAATCAACCTTT |
| 10 | TCCCCTAGGTCTGGAGACCTCTCTGTG |
| 11 | CAGTATTATAATGGAGAAGAGAGAGCAAAAGG AAACATTCTTGAACGATTCTCC |
| 12 | TGTGCCAGCAGCTTCCCTCTGGGGGGCGGGAGA GTGGATACGCAGTATTTT |
| 13 | LLLEATANL |
| 14 | MKSLRVLLVILWLQLSWVWSQQKEVEQNSGPLS VPEGAIASLNCTYSDRGSQSFFWYRQYSGKSPELIM SIYSNGDKEDGRFTAQLNKASQYVSLLIRDSQPSDS ATYLCAVTNAGKSTFGDGTTLTVKPN |
| 15 | MGFRLLCCVAFCLLGAGPVDSGVTQTPKHLITATGQ |
| | RVTLRCSPRSGDLSVYWYQQSLDQGLQFLIQYYNGE ERAKGNILERFSAQQFPDLHSELNLSSLELGDSALYF CASSFPLGGGRVDTQYFGPGTRLTVL |
| 16 | ATGAAATCCTTGAGAGTTTTACTAGTGATCCTGTGG CTTCAGTTGAGCTGGGTTTGGAGCCAACAGAAGGAG GTGGAGCAGAATTCTGGACCCCTCAGTGTTCCAGAG GGAGCCATTGCCTCTCTCAACTGCACTTACAGTGACC GAGGTTCCCAGTCCTTCTTCTGGTACAGACAATATTC TGGGAAAAGCCCTGAGTTGATAATGTCCATATACTCC AATGGTGACAAAGAAGATGGAAGGTTTACAGCACAG CTCAATAAAGCCAGCCAGTATGTTTCTCTGCTCATCAG AGACTCCCAGCCCAGTGATTCAGCCACCTACCTCTGTG CCGTTACGAATGCAGGCAAATCAACCTTTGGGGATGGG ACTACGCTCACTGTGAAGCCAAAT |
| 17 | ATGGGCTTCAGGCTCCTCTGCTGTGTGGCCTTTTGTCTC CTGGGAGCAGGCCCAGTGGATTCTGGAGTCACACAAAC CCCAAAGCACCTGATCACAGCAACTGGACAGCGAGTGA CGCTGAGATGCTCCCCTAGGTCTGGAGACCTCTCTGTGT ACTGGTACCAACAGAGCCTGGACCAGGGCCTCCAGTTC CTCATTCAGTATTATAATGGAGAAGAGAGAGCAAAAGG AAACATTCTTGAACGATTCTCCGCACAACAGTTCCCTGA CTTGCACTCTGAACTAAACCTGAGCTCTCTGGAGCTGGG GGACTCAGCTTTGTATTTCTGTGCCAGCAGCTTCCCTCTG GGGGGCGGGAGAGTGGATACGCAGTATTTTGGCCCAGGC ACCCGGCTGACAGTGCTC |
| 18 | IQNPEPAVYQLKDPRSQDSTLCLFTDFDSQINVPKTMESGTFIT DKTVLDMKAMDSKSNGAIAWSNQTSFTCQDIFKETNATYPSS DVPCDATLTEKSFETDMNLNFQNLSVMGLRILLLKVAGFNLL MTLRLWSS |
| 19 | EDLRNVTPPKVSLFEPSKAEIANKQKATLVCLARGFFPDHVEL SWWVNGKEVHSGVSTDPQAYKESNYSYCLSSRLRVSATFWH NPRNHFRCQVQFHGLSEEDKWPEGSPKPVTQNISAEAWGRAD CGITSASYHQGVLSATILYEILLGKATLYAVLVSGLVLMAMVK KKNS |
| 20 | IQNPEPAVYQLKDPRSQDSTLCLFTDFDSQINVPKTMESGTFIT DKCVLDMKAMDSKSNGAIAWSNQTSFTCQDIFKETNATYPSS DVPCDATLTEKSFETDMNLNFQNLSVMGLRILLLKVAGFNLL MTLRLWSS |
| 21 | EDLRNVTPPKVSLFEPSKAEIANKQKATLVCLARGFFPDHVELS WWVNGKEVHSGVCTDPQAYKESNYSYCLSSRLRVSATFWHNP RNHFRCQVQFHGLSEEDKWPEGSPKPVTQNISAEAWGRADCGIT SASYHQGVLSATILYEILLGKATLYAVLVSGLVLMAMVKKKNS |
| 22 | IQNPDPAVYQLRDSKSSDKSVCLFTDFDSQTNVSQSKDSDVYIT DKTVLDMRSMDFKSNSAVAWSNKSDFACANAFNNSIIPEDTFFP SPESSCDVKLVEKSFETDTNLNFQNLSVIGFRILLLKVAGFNLLM TLRLWSS |
| 23 | EDLKNVFPPEVAVFEPSEAEISHTQKATLVCLATGFFPDHVELSW |
| | WVNGKEVHSGVSTDPQPLKEQPALNDSRYCLSSRLRVSATFW QNPRNHFRCQVQFYGLSENDEWTQDRAKPVTQIVSAEAWGRAD CGFTSVSYQQGVLSATILYEILLGKATLYAVLVSALVLMAMVKR KDF |
| 24 | IQNPDPAVYQLRDSKSSDKSVCLFTDFDSQTNVSQSKDSDVYIT DKCVLDMRSMDFKSNSAVAWSNKSDFACANAFNNSIIPEDTFFPS PESSCDVKLVEKSFETDTNLNFQNLSVIGFRILLLKVAGFNLLMTL RLWSS |
| 25 | EDLKNVFPPEVAVFEPSEAEISHTQKATLVCLATGFFPDHVELSW WVNGKEVHSGVCTDPQPLKEQPALNDSRYCLSSRLRVSATFWQN PRNHFRCQVQFYGLSENDEWTQDRAKPVTQIVSAEAWGRADCGF TSVSYQQGVLSATILYEILLGKATLYAVLVSALVLMAMVKRKDF |
| 26 | DRGSQS |
| 27 | AVTNAGKST |
| 28 | SGDLS |
| 29 | YYNGEE |
| 30 | ASSFPLGGGRVDTQY |
| 31 | DKTVL |
| 32 | GVSTD |

### BRIEF DESCRIPTION OF THE DRAWINGS

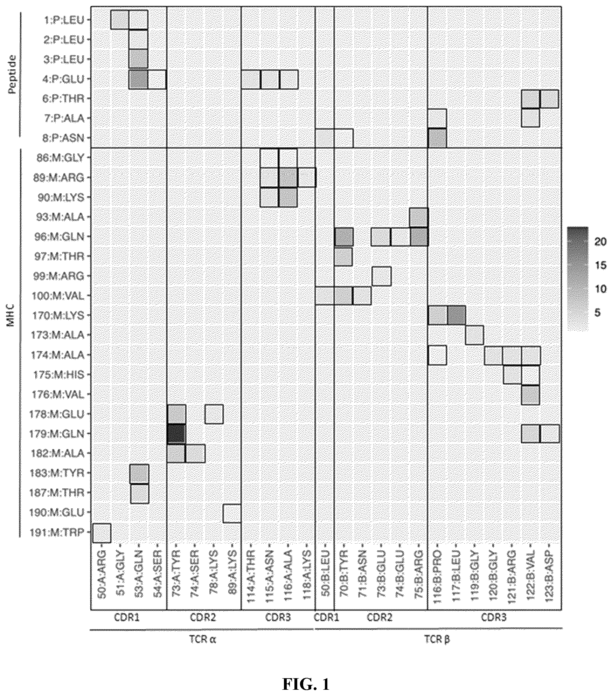

**Figure 1****:** Contacts between peptide-MHC and PR3-specific TCR (PR3_TCR) in the AlphaFold2 model. Atomics contacts are defined using a 5 Å cutoff and the number of contacts is summed on a per-residue basis. Grey levels correspond to the number of atomic contacts.

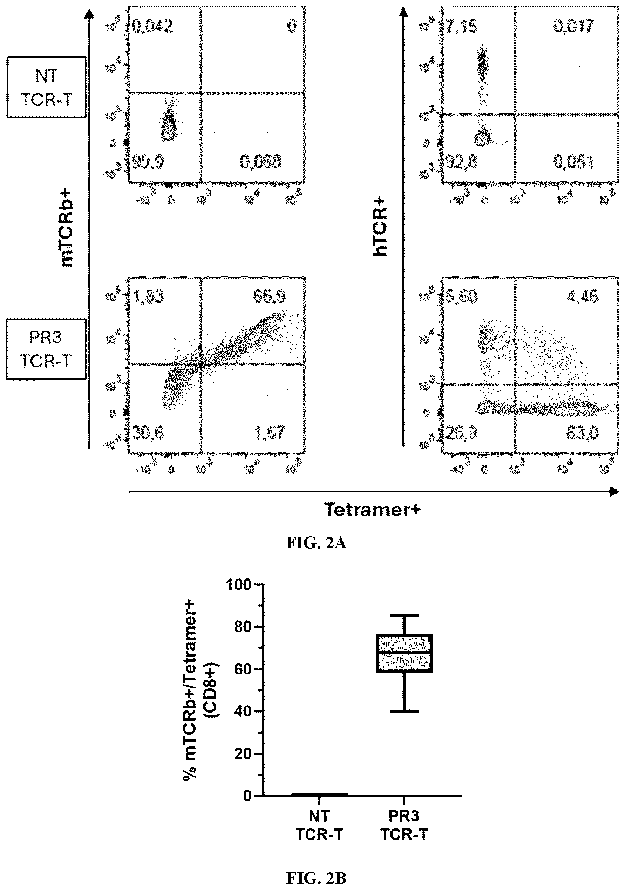

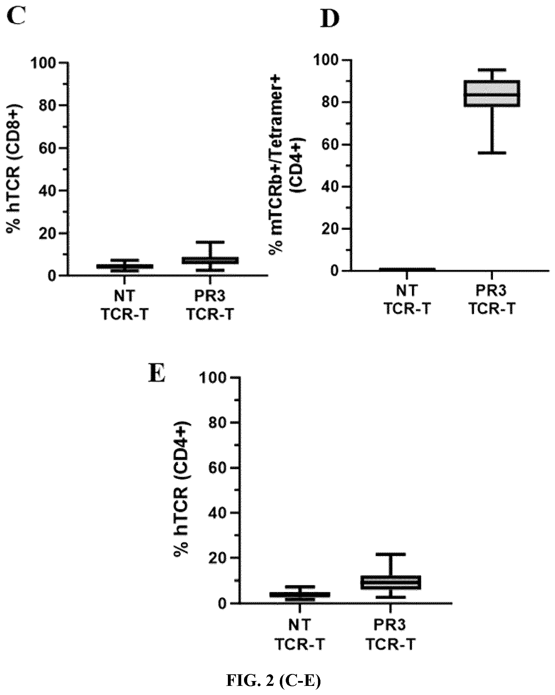

**Figure 2A****:** Representative plots of tetramer, mouse TCRβ (mTCRb) and human TCR (hTCR) staining among CD8+ T cells with a CRISPR-mediated knock-out of the endogenous TCR (CRISPR TCRαβ and either transduced with a PR3-specific TCR (PR3 TCR-T, lower row) or not transduced (NT TCR-T, upper row). **Figure 2B****:** Summary of the percentage of mTCRb/ Tetramer double-positive cells among CD8+ T cells 5 days after transduction of CRISPR TCRαβ T cells with PR3-specific TCR (PR3 TCR-T) and their control counterpart (NT TCR-T) (n=24). **Figure 2C****:** Summary of the percentage of hTCR positive cells among CD8+ T cells 5 days after transduction of CRISPR TCRαβ T cells with PR3-specific TCR (PR3 TCR-T) and their control counterpart (NT TCR-T) (n=24). **Figure 2D****:** Summary of the percentage of mTCRb/ Tetramer double-positive cells among CD4+ T cells 5 days after transduction of CRISPR TCRαβ T cells with PR3-specific TCR (PR3 TCR-T) and their control counterpart (NT TCR-T) (n=24). **Figure 2E****:** Summary of the percentage of hTCR positive cells among CD4+ T cells 5 days after transduction of CRISPR TCRαβ T cells with PR3-specific TCR (PR3 TCR-T) and their control counterpart (NT TCR-T) (n=24).

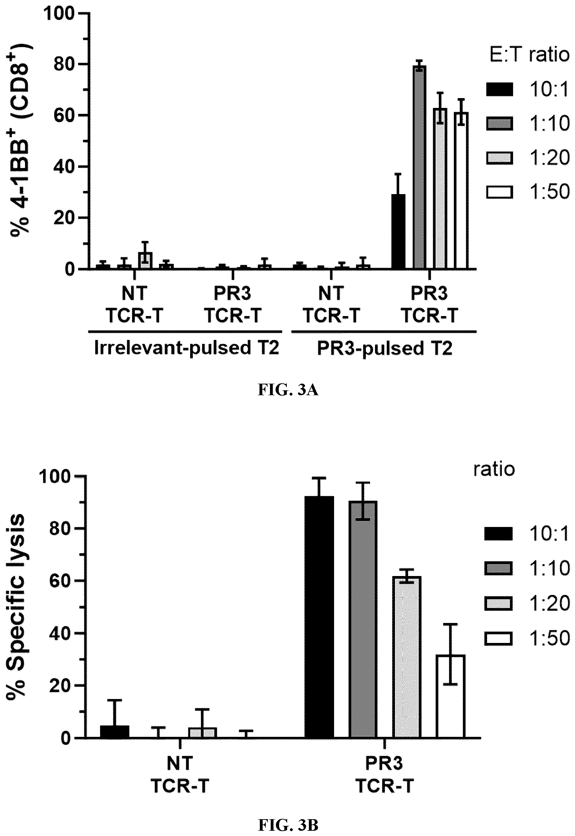

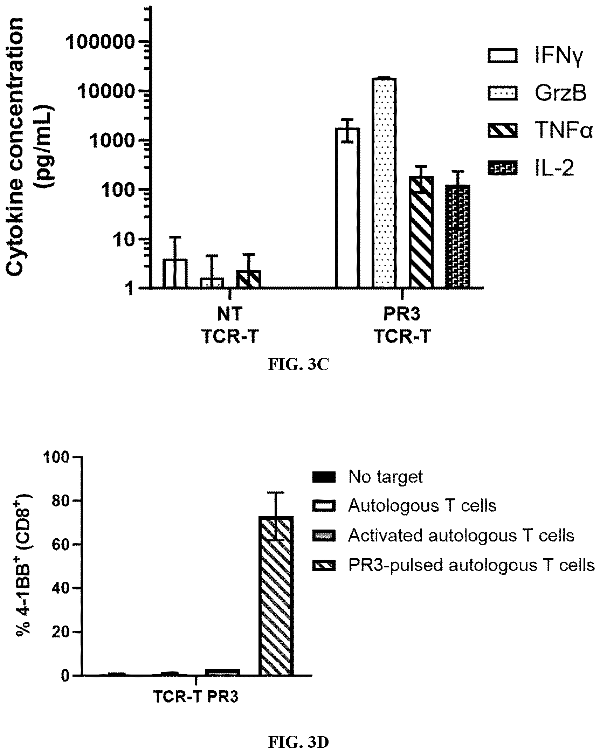

**Figure 3A****:** Representative graphic of the percentage of 4-1BB expression of PR3-specific TCR-T cells (PR3 TCR-T) or their control counterpart (NT TCR-T) after contact with T2 cells pulsed with irrelevant (irrelevant-pulsed T2) or specific peptide (PR3-specific T2), at different effector:target ratios (E:T ratio) (n=3). **Figure 3B****:** Representative graphic of the percentage of specific target lysis of PR3-specific TCR-T cells (PR3 TCR-T) or their control counterpart (NT TCR-T), at different effector:target ratios (E:T ratio). The specific target lysis is determined by dividing the percentage of specific peptide pulsed CFSE+ T2 cell death by the percentage of irrelevant (non-specific) peptide pulsed CFSE+ T2 cell death (n=3). **Figure 3C****:** Representative graphic of the cytokine concentration secreted by PR3-specific TCR-T cells (PR3 TCR-T) or their control counterpart (NT TCR-T), after contact with T2 cells pulsed with specific peptide (n=3). **Figure 3D****:** Representative graphic of the percentage of 4-1BB expression of PR3-specific TCR-T cells (PR3 TCR-T) alone, or after contact with autologous T cells, activated autologous T cells or autologous T cells pulsed with specific peptide (n=3).

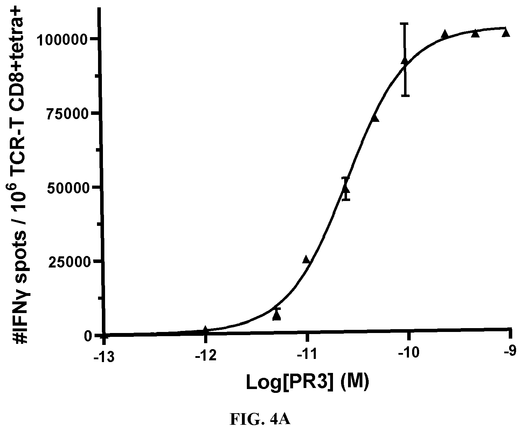

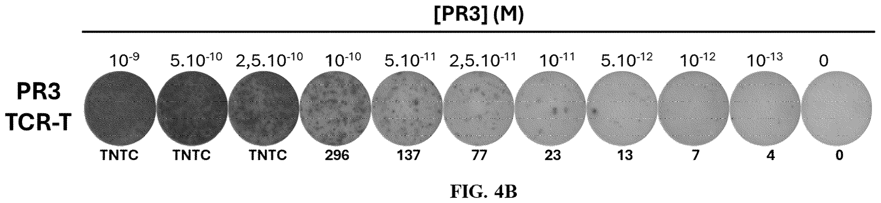

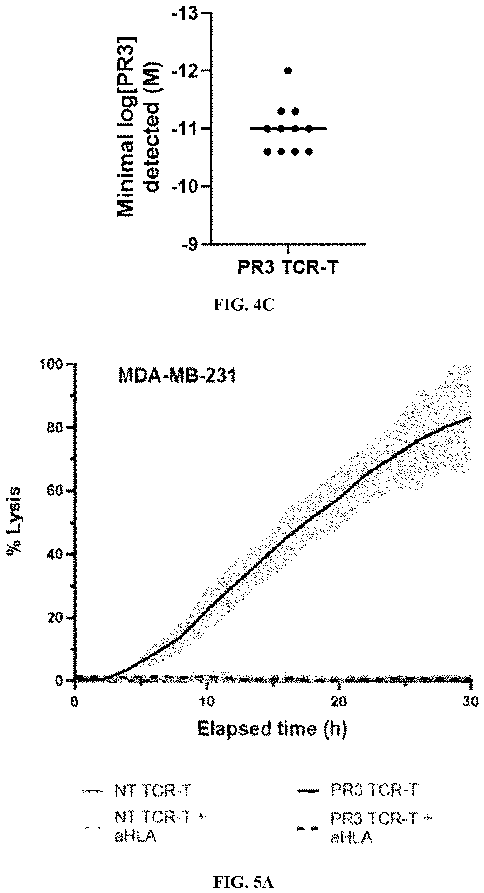

**Figure 4A****:** Representative graphic of the number of IFNγ spots secreted by PR3-specific TCR-T cells after contact with T2 cells pulsed with a decreasing concentration of PR3-specific peptide, using ELISpot. **Figure 4B****:** Representative ELISpot images of the number of IFNγ spots secreted by PR3-specific TCR-T cells after contact with T2 cells pulsed with a decreasing concentration of PR3-specific peptide, in duplicates (TNTC = too numerus to count). **Figure 4C****:** Summary of the PR3 minimal concentration detected by PR3-specific TCR-T cells (PR3 TCR-T), using IFNγ ELISpot (n=11).

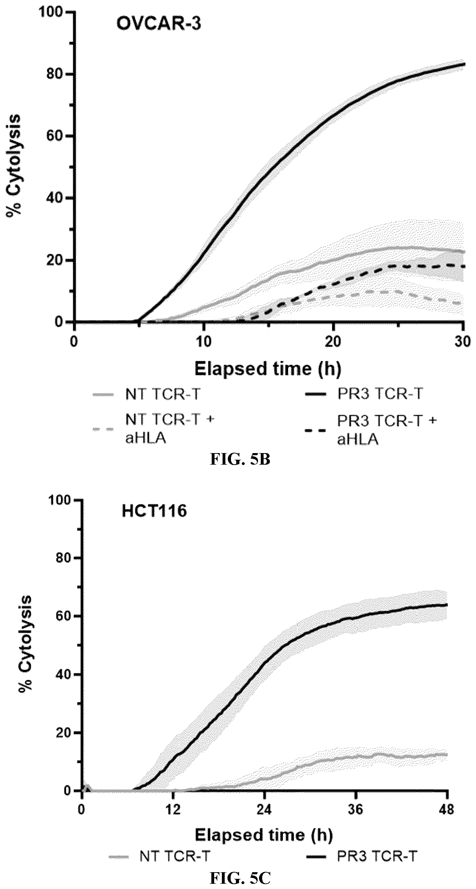

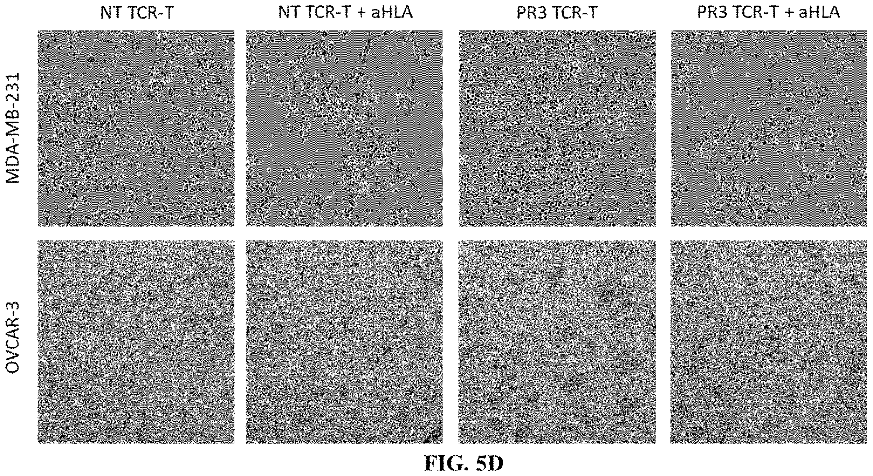

**Figure 5A****:** Representative curves of real-time cell death quantification (percentage of lysis) of MDA-MB-231 cell line co-cultured with PR3-specific TCR-T cells (PR3 TCR-T) or their negative counterpart (NT TCR-T) alone or with an anti-human MHC Class I antibody (aHLA). **Figure 5B****:** Representative curves of real-time cell death quantification (percentage of cytolysis) of OVCAR-3 cell line co-cultured with PR3-specific TCR-T cells (PR3 TCR-T) or their negative counterpart (NT TCR-T), alone or with an anti-human MHC Class I antibody (aHLA). **Figure 5C****:** Representative curves of real-time cell death quantification (percentage of cytolysis) of HCT116 cell line co-cultured with PR3-specific TCR-T cells (PR3 TCR-T) or their negative counterpart (NT TCR-T). **Figure 5D****:** Representative images at 30h of MDA-MB-231 cell line (upper row) or OVCAR-3 cell line (lower row) co-cultured with PR3-specific TCR-T cells (PR3 TCR-T) or their negative counterpart (NT TCR-T), alone or with an anti-human MHC Class I antibody (aHLA).

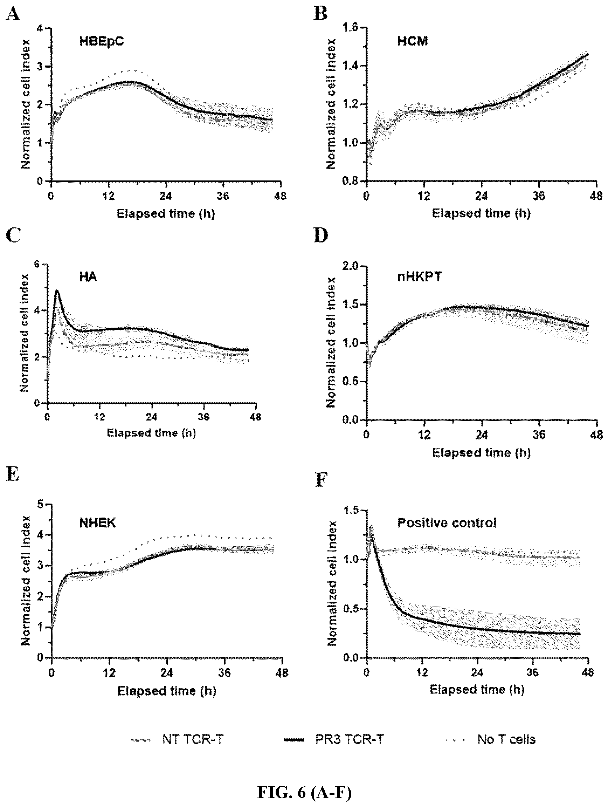

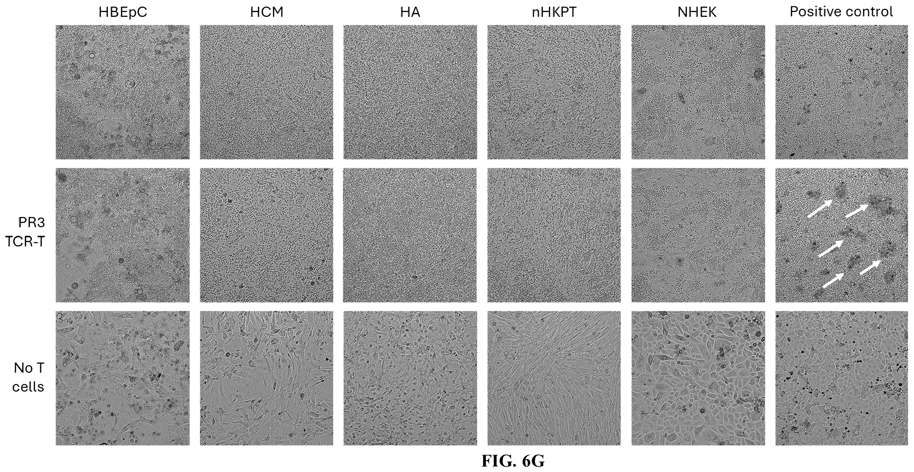

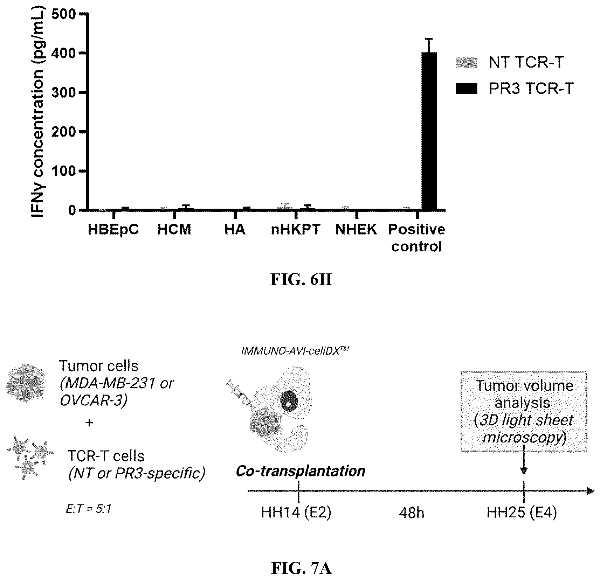

**Figures 6A-6F****:** Representative curves of the percentage of target cell death (normalized cell index) by xCELLigence of HLA-A2+ normal human primary cells alone (no T cells), co-cultured with PR3-specific TCR-T cells (PR3 TCR-T) or their negative counterpart (NT TCR-T). HBEpC = Human Bronchial Epithelial Cells **(A);** HCM = Human Cardiac Myocytes **(B);** HA = Human Astrocytes **(C);** nHKPT = Normal human kidney proximal tubule cells **(D);** NHEK = Normal Human Epithelial Keratinocytes **(E).** Positive control is endogenously PR3 expressing cell line OVCAR-3 **(F).** Cell index was normalized to the cell index at the time of TCR-T cells addition. **Figure 6G****:** Representative images at 48h of HLA-A2+ normal human primary cells alone (no T cells), co-cultured with PR3-specific TCR-T cells (PR3 TCR-T) or their negative counterpart (NT TCR-T), using xCELLigence. HBEpC = Human Bronchial Epithelial Cells ; HCM = Human Cardiac Myocytes ; NHEK = Normal Human Epithelial Keratinocytes ; nHKPT = Normal human kidney proximal tubule cells ; HA = Human Astrocytes. Positive control is the cell line OVCAR-3 endogenously expressing PR3. White arrows indicate T cell clusters reminiscent of T cell activation and target killing. **Figure 6H****:** Representative graphic of IFNγ concentrations produced by PR3-specific TCR-T cells (PR3 TCR-T) or their negative counterpart (NT TCR-T) after 48h co-culture with either HLA-A2+ normal human primary cells. HBEpC = Human Bronchial Epithelial Cells ; HCM = Human Cardiac Myocytes ; NHEK = Normal Human Epithelial Keratinocytes ; nHKPT = Normal human kidney proximal tubule cells ; HA = Human Astrocytes (n=3).

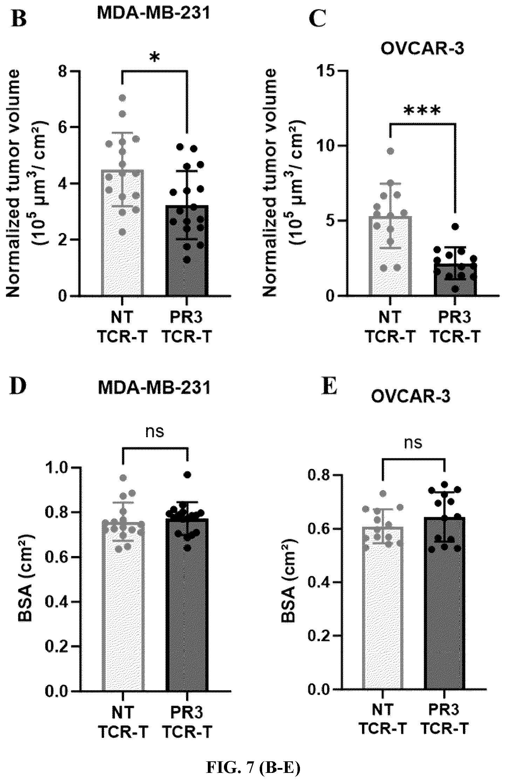

**Figure 7A****:** Schematic representation of the *in vivo* protocol using immuno-AVI-cellDXTM model to evaluate the anti-tumoral efficacy of TCR-T cells. Fluorescently labelled tumor cell lines (MDA-MB-231 or OVCAR-3) are co-injected with fluorescently labelled PR3-specific TCR-T cells (PR3 TCR-T) or their negative counterpart (NT TCR-T) in the immuno-AVI-cellDXTM embryo model at stage HH14 (E2). After 48h, the tumor volume is analyzed using 3D light sheet microscopy. **Figure 7B****:** Representative graphic of normalized tumor volume of MDA-MB-231 tumor cell line co-injected with PR3-specific TCR-T cells (PR3 TCR-T, n=17) or their negative counterpart (NT TCR-T, n=16) in the immuno-AVI-cellDXTM embryo model. Normalized tumor volume is determined by dividing raw tumor volume by the body surface area (BSA) for each single viable embryo. * = p < 0.05. **Figure 7C****:** Representative graphic of normalized tumor volume of OVCAR-3 tumor cell line co-injected with PR3-specific TCR-T cells (PR3 TCR-T, n=13) or their negative counterpart (NT TCR-T, n=13) in the immuno-AVI-cellDXTM embryo model. Normalized tumor volume is determined by dividing raw tumor volume by the body surface area (BSA) for each single viable embryo. *** = p < 0.001. **Figure 7D****:** Representative graphic of body surface area (BSA) of MDA-MB-231 tumor cell line co-injected with PR3-specific TCR-T cells (PR3 TCR-T, n=17) or their negative counterpart (NT TCR-T, n=16) in the immuno-AVI-cellDXTM embryo model. ns = non significative. **Figure 7E****:** Representative graphic of body surface area (BSA) of OVCAR-3 tumor cell line co-inj ected with PR3-specific TCR-T cells (PR3 TCR-T, n=13) or their negative counterpart (NT TCR-T, n=13) in the immuno-AVI-cellDXTM embryo model. ns = non significative.

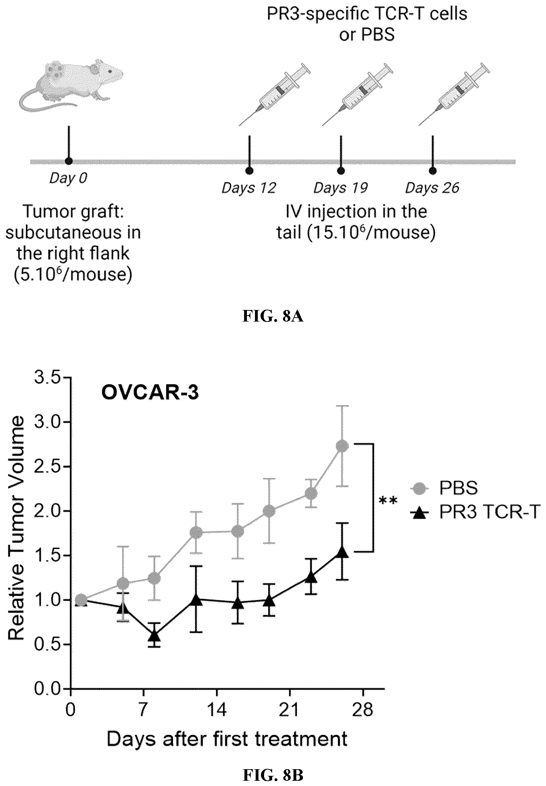

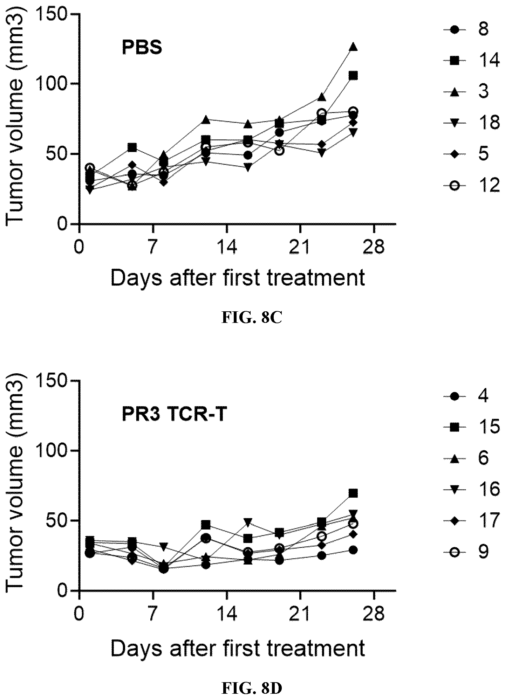

**Figure 8A****:** Schematic representation of the *in vivo* protocol to evaluate anti-tumoral activity of PR3-specific TCR-T cells against OVCAR-3 cell lines. 5 × 10⁶ tumor cells were injected subcutaneously in 10-12 weeks old mice. 12 days after engraftment, 15 ×10⁶ PR3-specific TCR-T cells (n=6) or PBS (n=6) were injected intravenously (IV). A second and third injection were performed 7 days and 14 days after the first injection respectively. **Figure 8B****:** Representative graph of relative tumor volume (RTV) of OVCAR-3 bearing tumors mice treated with PR3-specific TCR-T cells (PR3 TCR-T, n=6) or PBS as a control (n=6), between the day of the first injection and 26 days after. The relative tumor volume is normalized to the initial tumor volume at time of treatment. ** = p < 0.01. **Figure 8C****:** Representative graph of the tumor volume of each OVCAR-3 bearing tumor mice treated with PBS as a control (n=6), between the day of the first injection and 26 days after. **Figure 8D****:** Representative graph of the tumor volume of each OVCAR-3 bearing tumor mice treated with PR3-specific TCR-T cells (PR3 TCR-T, n=6), between the day of the first injection and 26 days after.

### EXAMPLES

The present invention is further illustrated by the following examples.

### Example 1:

### Single cell TCR sequencing

PR3-specific CD8+ T cells were first sorted as a single cell suspension using BD FACSAria^{™} Cell Sorter. Then, single cell TCR sequencing was performed using the Chromium Single Cell Immune Profiling from 10X Genomics, as described in the manufacturer's protocol. Briefly, thousands of gel beads-in-emulsion (GEMs) containing a single T cell, the functionalized gel bead and RT reagents in solution were partitioning by the Chromium system. 10X Next GEM Technology samples a pool of ~ 750 000 10X barcodes to separately index each cell's transcriptome. Single cell GEMs undergo reverse transcriptase to generate 10X barcoded cDNA. All generated cDNA from individual cells share a common 10X barcode. The partitions in the emulsion are broken open and all barcodes cDNAs from all GEMs are mixed. V(D)J and gene expression libraries are generated and sequenced and 10x Barcodes are used to associate individual reads back to the individual partitions. The most frequent clonotype was selected for TCR-T cells generation.

Table 1 shows the V and J alleles of the TCRα and TCRβ chains of the PR3-specific TCR (PR3_TCR), determined by single cell TCR sequencing.

**Table 1**

| | TCRα | | TCRβ | |
|---|---|---|---|---|
| | V allele | J allele | V allele | J allele |
| PR3_TCR | TRAV12-2 | TRAJ27 | TRBV9 | TRBJ2-3 |

CDR1, CDR2 and CDR3 sequences of the TCRα and TCRβ chains of the PR3-specific TCR were predicted using the TCRpMHCmodels - 1.0 (DTU Health Tech). The CDR1-3 sequences are indicated in the Table 2.

**Table 2**

| | **Nucleotide sequence** | **Amino acid sequence** |
|---|---|---|
| **TCRα chain** | | |
| **CDR1** | SEQ ID NO: 7 | SEQ ID NO: 1 |
| **CDR2** | SEQ ID NO: 8 | SEQ ID NO: 2 |
| **CDR3** | SEQ ID NO: 9 | SEQ ID NO: 3 |

| **TCRβ chain** | | |
|---|---|---|
| **CDR1** | SEQ ID NO: 10 | SEQ ID NO: 4 |
| **CDR2** | SEQ ID NO: 11 | SEQ ID NO: 5 |
| **CDR3** | SEQ ID NO: 12 | SEQ ID NO: 6 |

| | | |
|---|---|---|
| *Contact prediction between TCR and MHC-peptide* | | |

3D models were predicted with AlphaFold2. The prediction was run online with ColabFold v1.5.3 and the following options: no templates, model=alphafold2_multimer_v3, number of recycling=3, pair mode=unpaired_paired, msa_mode=mmseqs2_uniref_env. Five models were generated and ranked by the multimer composite score (0.8*ipTM +0.2pTM). Only the best model was minimized and kept for further analysis.

Results in Figure 1 show the predicted contacts between peptide-MHC (pMHC) and the PR3-specific TCR, using AlphaFold2 model. These TCR-pMHC interactions concur with previous studies showing that, whereas CDR1 and CDR3 loops of both TCRα and TCRβ chains interact with both the MHC and the peptide, the CDR2 loops interact mostly with the MHC molecule.

### Peptide synthesis

Peptides were synthetized (ThermoFisher Scientific) and their identities were confirmed by mass spectrometry by the seller. Purity > 90% was expected and determined by high-performance liquid chromatography. Lyophilized peptides were dissolved in deionized water < 5% DMSO, aliquoted and conserved at -20°C until use.

### Lentivirus production

PR3-specific TCR was previously identified using single cell TCR sequencing of PR3-specific CD8+ T cells. PR3 TCR sequence was optimized by replacing human constant regions by a murine one. PR3 TCR coding plasmid and other packaging plasmids necessary for virion production (psPAX2 and pMDG) were purchased at R&D Biotech (Besançon). 293T cell line (ATCC) were cultured in DMEM media supplemented with 10% Fetal Calf Serm (FCS), 1% Penicillin/Streptomycin (PS) and 0,1mM of MEM non-essential amino acids (Invitrogen). Cells were seeded in 100 mm dishes to obtain a confluence of 80-90% the day of the transfection. Cells were transfected using the Calcium Phosphatase transfection kit (Invitrogen) according to the manufacturing protocol. Plasmid solutions were used at a ratio of 1:1.5:3.6 (psPAX2 : pMDG : TCR coding plasmid). The next day, cell media was replaced by a media without serum. 48h and 72h after transfection, the supernatants containing the lentivirus were collected, pooled together and centrifuged for 16h at 3000g, 4°C. Lentivirus production media was also centrifuged in the same condition as a control for transduction. Lentivirus or media were aliquoted and stocked at -80°C.

### T cells transduction

PBMCs were obtained by Ficoll density gradient centrifugation of blood from healthy donors ("Etablissement Français du Sang", EFS, Lyon). PBMCs were rapidly thawed at 37°C, extensively washed and resuspended in RPMI + 8% AB serum (sAB) + 1% PS media. PBMCs were incubated overnight in a 37°C incubator. The next day, PBMCs were collected and their viability was assessed. In order to isolate and activate T cells, PBMCs were incubated with anti-CD3 and anti-CD28 magnetic beads (Dynabeads^{™} Human T-Expander CD3/CD28, Gibco) for 20min at room temperature on agitation. After magnetic sorting, activated T cells were cultured in X-VIVO 15 (Lonza) 8% sAB + 1% PS supplemented with 10ng/ml of human recombinant IL-7 and IL-15 for 48h in a 37°C incubator. Endogenous TCR was deleted using CRISPR-Cas 9 technology. Briefly, ribonucleoprotein (RNP) complex was formed by hybridization of CRISPR RNAs (crRNAs) targeting the TRAC and TRBC, trans-activating crispr RNA (tracrRNA) and Cas9 protein. RNPs were electroporate into T cells using the Neon transfection system (Thermo Fisher Scientific). Then, CRISPR TCRαβ T cells were seeded in a previously RetroNectin-(Takara) coated plate (1µg/ml) and transduced using either with lentivirus coding for the PR3-specific TCR or with media as a control (non-transduced condition, NT). After 4 days, cells were collected and the transduction efficacy was evaluated by multiparametric flow analysis.

### Multiparametric flow cytometry

Tetramer were produced by incubated peptide specific biotinylated monomer (P2R Facility, Nantes, France) with PE Streptavidin antibody (Biolegend) on a rotary shaker for 1h at room temperature.

For the staining, between 0,5 and 1.10⁶ cells were put in polypropylene tubes and washed with FACS buffer. Cells were stained with a mix of antibodies during 1h at 4°C in the dark. The mix was composed of PR3-PE-Tetramer at 10 µg/ml, 1/400 diluted Zombi Near Infra-Red (NIR), anti-CD4 BV605, anti-CD8 FITC, anti-mouse TCRb (mTCRb) BV421 and anti-human TCR (hTCR) APC antibodies (Biolegend). Cells were then washed twice with FACS buffer (1X PBS, 2mM EDTA, 1% FCS) and resuspended in FACS buffer for Flow Cytometry analysis (FACS Fortessa BD).

Results in Figure 2A show a representative staining of CD8+ TCR-T cells with either Tetramer and mTCRb antibodies (left panel) or Tetramer and hTCR antibodies (right panel). On average, 66.9% of CD8+ PR3-specific TCR-T cells with CRISPR TCRαβ (Figure 2B) and 82.6% of CD4+ PR3-specific TCR-T cells with CRISPR TCRαβ (Figure 2D) expressed the PR3-exogenous TCR compared to 0% for CD8+ or CD4+ NT TCR-T cells with CRISPR TCRαβ (Figures 2B and 2D), as shown by the mTCRb+/Tetramer+ stained cells. 7.2% of CD8+ PR3-specific TCR-T cells with CRISPR TCRαβ have a remaining hTCR compared to 4.4% for CD8+ NT TCR-T cells with CRISPR TCRαβ (Figure 2C). 9.4% of CD4+ PR3-specific TCR-T cells with CRISPR TCRαβ have a remaining hTCR compared to 4.1% for CD4+ NT TCR-T cells with CRISPR TCRαβ (Figure 2E).

### Functional analysis

T2 cell line is a lymphoblast cell line deficient in the transporter associated antigen processing (TAP) protein, and therefore cannot present endogenous peptides on the class I MHC but can be used to monitor the Cytotoxic T Lymphocyte (CTL) response to an exogenous antigen of interest in a non-competitive environment.

T2 cells were first stained with CFSE cell division tracker kit (Biolegend) for 13 min at 37°C and then washed 3 times. CFSE-stained T2 cells were pulsed with irrelevant (non-specific) or specific peptide for 2 hours at 37°C. After extensive washed, CFSE+ pulsed T2 cells were resuspended in X-VIVO 15 + 8% sAB + 1% PS. In parallel, resting was performed on TCR-T cells. First, TCR-T cells were resuspended in X-VIVO 15 8% sAB + 1% PS supplemented with 1ng/ml of human recombinant IL-7 and IL-15 overnight at 37°C. The next day, TCR-T cells were resuspended in their corresponding medium at 37°C for 2h without cytokine supplementation. Then, CFSE+ pulsed T2 cells and T cells were co-cultured at different E:T (effector:target) ratios in 96-well U-bottom plate in duplicate. After 24h, supernatant was collected for further ELISA analysis and cells were pooled in V-well plate and washed with FACS buffer. For each condition, a mix of antibody was added containing Zombi NIR (dil 1/400), anti-CD3 BV421, anti-CD8 APC and anti-human CD137 (4-1BB) Pe-Dazzle594 (Biolegend) antibodies for 30 min at 4°C in the dark. Cells were washed and resuspend in FACS buffer before Flow Cytometry analysis (FACS Fortessa BD).

Figure 3A shows the percentage of 4-1BB expressing cells within peptide-specific or unspecific CD8+ TCR-T cells after contact with irrelevant or specific peptide-pulsed T2 cells. Figure 3B shows the percentage of T2 cell death after contact with peptide-specific CD8+ TCR-T cells or their unspecific counterpart. These results show a specific activation (Figure 3A) and a specific killing (Figure 3B) of PR3-specific CD8+ TCR-T cells upon specific antigenic stimulation.

For ELISA analysis, IFN gamma Human Uncoated ELISA Kit (Invivogen), TNF alpha Human Uncoated ELISA Kit (Invivogen), Human IL-2 DuoSet ELISA (Bio-Techne) and Human Granzyme B DuoSet ELISA (R&D Systems) were used according to the manufacturer's instructions. Supernatants were diluted at 1/5 for the analysis.

Results on Figure 3C show cytokine secretion quantification of NT or PR3-specific TCR-T cells after contact with irrelevant or specific peptide-pulsed T2 cells. Concomitantly with previous results, there was a specific secretion of IFNγ, TNF, IL-2 and Granzyme B of PR3-specific TCR-T cells upon specific antigenic stimulation.

### Functional avidity by IFNy ELISpot

T2 cells are pulsed with a decreasing concentration of PR3-specific peptide for 2 hours at 37°C. After extensive washes, pulsed T2 cells were resuspended in X-VIVO 15 8% sAB + 1% PS. TCR-T cells were rested first overnight at 37°C in their corresponding medium supplemented with 1ng/ml of human recombinant IL-7 and IL-15, and the next day for 2h at 37°C in their corresponding medium without cytokine supplementation. Prior to co-culture, MultiScreen HTS IP Filter Plate, 0.45 µm Pore Size, (Millipore) were coated according to Human IFNγ ELISpot Set (Diaclone) manufacturer's instructions. Pulsed T2 cells and PR3-specific TCR-T cells are co-cultured at 1:10 effector: target ratio in duplicates for 18h at 37°C. Revelation of the IFNγ ELISpot was performed according to the manufacturer's instructions. Revelation plate was read on an ImmunoSpot S6 ULTIMATE UV image analyzer and analyzed with the ImmunoSpot analysis software.

Results in Figure 4 show the numbers of IFNγ spot detected when PR3-specific TCR-T cells were co-cultured with a decreasing concentration of specific pulsed T2 cells. As shown in the graph (Figure 4A) and the images (Figure 4B), PR3-specific TCR-T cells can detect up to 1.10⁻¹¹ M of PR3 peptide. Figure 4C represents a summary of multiple PR3-specific TCR-T cells IFNγ ELISpot, with an average of minimal detected PR3-peptide concentration of 1.10⁻¹¹ M. This demonstrates the high functional avidity of the PR3-specific TCR-T cells.

### In vitro cytotoxicity and safety of PR3-specific T cells against tumor cell lines using live imaging.

In order to monitor tumor cell death in real-time, we performed an immune cell killing assay using the either IncuCyte (Sartorius) or xCELLigence Real time Cell Analysis (Agilent) technologies. Incucyte technology enables the measurement of Fluorescence and HD phase cell imaging in real time. xCELLigence technology enables the measurement of both live cell imaging and cellular impedance of adherent cells at the same time. Cellular impedance reflects the morphology change of adherent cells. For example, cell proliferation induces an increase of cellular impedance. In contrast, cell death induces a decrease of cellular impedance. Impedance is only influenced by adherent cells and not suspension cells (T cells for example).

For *in vitro* cytotoxicity analysis, tumor cell lines used as target were breast cancer cell line MDA-MB-231, ovarian cancer cell line OVCAR-3 and colon cancer cell line HCT116. MDA-MB-231 cell line was transduced to express a nuclear mCherry marker. As a positive control, cell lines were pulsed with the specific peptide to artificially present the target epitope on class I MHC molecules. The day before the experiment, tumor cell lines were seeded in 96-well flat-bottom plate for Incucyte assay, and E-Plate VIEW 96 (Agilent) for xCELLigence assay. For TCR-T cells, a resting was performed overnight with IL-7 and IL-15 cytokines supplementation at 1 ng/ml and a 2h resting the next day without cytokine. According to conditions, tumor cell lines were either pulsed with the specific peptide at 10 µg/ml for 2h and then washed or incubated with 50 µg of blocking HLA-A2 antibody (InVivoMab anti-human MHC Class I) was added. TCR-T cells were added in corresponding wells (CD8+ T cells: tumor cell lines ratio 2:1). Finally, Incucyte^{®} Cytotox Green for Counting Dead Cells (Sartorius) was added to reach a final concentration of 250 nM per well. This reagent enters the cells when the plasma membrane integrity is compromised, yielding a 100-1000-fold increase in fluorescence upon binding to deoxyribonucleic acid (DNA). A 30 to 48-hour live imaging was performed at 37°C 5% CO2 with Incucyte Zoom or xCELLigence RTCA eSight. For the xCELLigence analysis, tumor cell death was monitored using impedance, expressed as an arbitrary unit called cell index. Percentage of cytolysis was calculated using the immunotherapy module of RTCA eSight Software. For Incucyte analysis, tumor cell death was monitored by evaluating the number of dead target cells (green+red fluorescence) divided by the number of target cells (red fluoresence) over time. Percentage of specific lysis was calculated according to the following formula: (TCR-T induced target cell death - spontaneous target cell death)/(Average (PR3-TCR-T-induced pulsed target cell death at 48h) - spontaneous target cell death) × 100.

Results on Figure 5 show the tumor cell death kinetics after co-culture with PR3-specific TCR-T cells or with NT-TCR-T cells for MDA-MB-231 (Figure 5A), OVCAR-3 (Figure 5B) or HCT116 cell lines (Figure 5C), as well as a representative image of MDA-MB-231 and OVCAR-3 cell lines condition at 30h (Figure 5D). These results show an increase of target cell death after co-culture with PR3-specific TCR-T cells compared to NT-TCR-T cells for each tumor cell line condition. Interestingly, specific target cell death was decreased or inhibited when HLA-A2 was blocked for MDA-MB-231 and OVCAR-3 cell line, showing that the cytotoxic effect of PR3 specific TCR-T cells is mediated by the recognition of the PR3 epitope presented by HLA class I molecules at the surface of the tumor cells (HLA class I restricted lysis).

For safety analysis, the cytotoxicity of PR3-specific TCR-T cells was evaluated by xCELLigence analysis as described above on a panel of HLA-A2+ normal human primary cells (HBEpC = Human Bronchial Epithelial Cells ; HCM = Human Cardiac Myocytes ; NHEK = Normal Human Epithelial Keratinocytes ; NhKPT = Normal human kidney proximal tubule cells ; HA = Human Astrocytes). Positive control is endogenously PR3 expressing cell line OVCAR-3.

Results on Figure 6 show the normalized cell index of normal primary cells over time, alone or co-cultured with PR3-specific TCR-T cells or NT TCR-T cells (Figures 6A-F) as well as a representative image at 48h (Figure 6G). As shown in Figures 6A-F, there is no difference in cell index between normal primary cells alone of co-culture with PR3-speecific TCR-T cells, demonstrating that PR3-specific TCR-T cells do not kill normal cells. Additionally, IPNγ cytokine was evaluated on the supernatant at the end of the co-culture, using ELISA assay (Figure 6H). No IFNγ was detected when PR3-pecific TCR-T cells were co-cultured with any of the normal primary cells, showing that PR3-specific TCR-T cells are not activated when co-cultured with normal primary cells, further supporting the lack of recognition of normal cells by PR3-specific TCR-T cells.

Altogether these results demonstrate that PR3-specific TCR-T cells specifically recognize and kill tumor cells but spare normal cells.

### In vivo cytotoxicity of PR3-specific TCR-T cells against tumor cell lines using immuno-AVI-cellDXTM model of TNBC.

*In vivo* anti-tumoral efficacy of PR3-specific TCR-T cells was performed by Oncofactory (Lyon, France). Briefly, fluorescently labelled MDA-MB-231 or OVCAR-3 tumor cell lines are co-injected with fluorescently labelled PR3-specific TCR-T cells or NT TCR-T cells in the immuno-AVI-cellDXTM embryo model at stage HH14 (E2) (effector: target ratio 5:1) (n=25). 48h post-engraftment, quantitative analysis of the tumor volume in each single viable embryo using 3D light sheet microscopy was performed (Figure 7A).

Results in Figure 7 show the normalized tumor volume and the body surface area (BSA) of MDA-MB-231 (Figures 7B and 7D) or OVCAR-3 (Figures 7C and 7E) bearing immuno-AVI-cellDXTM embryo model, co-injected with PR3-specific TCR-T cells or NT TCR-T cells. These results show that PR3-specific TCR-T cells co-transplanted with MDA-MB-231 or OVCAR-3 have an anti-tumoral activity, inducing a significant decrease in tumor volume compared to control experimental group (treated with NT TCR-T cells).

### Subcutaneous tumor xenografts

OVCAR-3 cells were amplified, trypsinized, counted and resuspended in 50% Matrigel, 50% 1X PBS at 5.106 per 100µL. 100µL of this solution was injected subcutaneously on the right flank of 6 NSG mice (Charles River) for each condition (12 mice total). Animals were housed and monitored in accordance with the protocol validated by the Centre Leon Bérard IACUC committee. Mice were monitored daily, weighed twice a week and tumor size was measured three time a week using a caliper. Animals were euthanized before the tumor volume reaches 1500mm³, in agreement with the French law, as determined by the formula V = L × l2 /2.

### Tail vein injection of TCR-T cells and assessment of tumor volume

PR3-specific TCR-T cells were amplified up to 9 days after the first activation step in X-VIVO 15 + 8% sAB + 1% PS supplemented with IL-7 and IL-15 at 10 ng/mL as described above. Cells were resuspended at 15.10⁶cells / 100µL in 1XPBS and injected in the tail vein of 10 to 12-week-old NSG mice 12 days after tumor engraftment. Tumor growth was monitor with a caliper every 2 to 3 days.

Results in Figure 8A show a schematic representation of the protocol for *in vivo* assessment of an-tumoral activity of PR3-specific TCR-T cells. Figure 8B shows the evolution of the relative tumor volume of OVCAR-3 tumor bearing mice treated with PR3-specific TCR-T cells or PBS 1X as a control. These results show a decrease of the tumor growth in mice treated with PR3-specific TCR-T cells. Figures 8C and 8D represent the evolution of the individual tumor volume for the mice treated with PR3-specific TCR-T cells or PBS as a control, respectively.

Altogether, these experiments show that PR3-specific TCR-T cells specifically recognize and are functional against target cells presenting the cognate peptide. They also show specific cytotoxic activity *in vitro* and *in vivo* against tumor cell lines expressing endogenously the PR3 peptide (MDA-MB-231, OVCAR-3 and HCT116), with no cytotoxic effect against normal human primary cells.

## Claims

1. An isolated T cell receptor (TCR) binding specifically to an epitope derived from non-canonical initiation and/or termination of translation of a gene of the *MYC* family, preferably *C-MYC.*

2. The isolated TCR according to claim **1,** wherein the epitope comprises or consists of a sequence with SEQ ID NO: 13.

3. The isolated TCR according to any one of claims **1** to **2,** wherein the TCR comprises a α chain and a β chain, wherein the variable region of the α chain binds at least one of the following residues of SEQ ID NO: 13: Leu1, Leu2, Leu3, Glu4, and wherein the variable region of the β chain binds at least one of the following residues of SEQ ID NO: 13: Thr6, Ala7, Asn8.

4. The isolated TCR according to any one of claims **1** to **3,** wherein the TCR comprises a α chain and a β chain,
- wherein the variable region of the α chain comprises at least one of the following CDRs:
• CDR1 comprising or consisting of SEQ ID NO: 26,
• CDR2 comprising or consisting of SEQ ID NO: 2, and/or
• CDR3 comprising or consisting of SEQ ID NO: 27, or variants thereof having at least 90% of identity with SEQ ID NOs: 26, 2 and 27, and
- wherein the variable region of the β chain comprises at least one of the following CDRs:
• CDR1 comprising or consisting of SEQ ID NO: 28,
• CDR2 comprising or consisting of SEQ ID NO: 29, and/or
• CDR3 comprising or consisting of SEQ ID NO: 30, or variants thereof having at least 90% of identity with SEQ ID NOs: 28-30.

5. The isolated TCR according to any one of claims **1** to **4,** wherein the TCR comprises a α chain and a β chain,
- wherein the variable region of the α chain comprises the 3 following CDRs:
• CDR1 comprising or consisting of SEQ ID NO: 26,
• CDR2 comprising or consisting of SEQ ID NO: 2, and
• CDR3 comprising or consisting of SEQ ID NO: 27, or variants thereof having at least 90% of identity with SEQ ID NOs: 26, 2 and 27, and
- wherein the variable region of the β chain comprises the 3 following CDRs:
• CDR1 comprising or consisting of SEQ ID NO: 28,
• CDR2 comprising or consisting of SEQ ID NO: 29, and
• CDR3 comprising or consisting of SEQ ID NO: 30, or variants thereof having at least 90% of identity with SEQ ID NOs: 28-30.

6. The isolated TCR according to any one of claims **1** to **5,** wherein the TCR comprises an α chain and a β chain,
- wherein the variable region of the α chain comprises the 3 following CDRs:
• CDR1 consisting of SEQ ID NO: 1,
• CDR2 consisting of SEQ ID NO: 2, and
• CDR3 consisting of SEQ ID NO: 3, and
- wherein the variable region of the β chain comprises the 3 following CDRs:
• CDR1 consisting of SEQ ID NO: 4,
• CDR2 consisting of SEQ ID NO: 5, and
• CDR3 consisting of SEQ ID NO: 6.

7. The isolated TCR according to any one of claims **1** to **6,** wherein the TCR comprises a α chain and a β chain,
- wherein the variable region of the α chain comprises or consists of the sequence with SEQ ID NO: 14, or a variant thereof having at least 90% of identity with SEQ ID NO: 14, and
- wherein the variable region of the β chain comprises or consists of the sequence with SEQ ID NO: 15, or a variant thereof having at least 90% of identity with SEQ ID NO: 15.

8. The isolated TCR according to any one of claims **1** to **7,** comprising murine constant regions.

9. A nucleic acid encoding the isolated TCR according to any one of claims **1** to **8.**

10. The nucleic acid according to claim **9,** wherein the nucleic acid comprises:
- a sequence encoding the variable region of the α chain of the TCR with SEQ ID NO: 16, or a variant thereof having at least 70% of identity with SEQ ID NO: 16, and
- a sequence encoding the variable region of the β chain of the TCR with SEQ ID NO: 17, or a variant thereof having at least 70% of identity with SEQ ID NO: 17.

11. A vector comprising the nucleic acid according to any one of claims **9** to **10.**

12. A cell comprising the isolated TCR according to any one of claims **1** to **8,** the nucleic acid according to any one of claims **9** to **10,** or the vector according to claim **11,** wherein preferably the cell is a T-cell, more preferably wherein the T cell is modified for silencing or genetically knocking out endogenous TCR chains.

13. The isolated TCR, the nucleic acid, the vector, or the cell according to any one of claims **1** to **12,** for use as a medicament.

14. The isolated TCR, the nucleic acid, the vector, or the cell according to any one of claims **1** to **12,** for use in treating a cancer in a subject in need thereof.

15. The isolated TCR, the nucleic acid, the vector, or the cell for use according to claim **14,** wherein the cancer is selected from the group comprising or consisting of breast cancer, including triple negative breast cancer, ovarian cancer, melanoma, sarcoma, teratocarcinoma, colon cancer, prostate cancer, bladder cancer, lung cancer, including non-small cell lung carcinoma and small cell lung carcinoma, head and neck cancer, colorectal cancer, glioblastoma, leukemias, lymphomas and other solid tumors and hematological malignancies.
